# EUROPEAN PATENT APPLICATION

(11) **EP 2 147 927 A2**
(43) Date of publication of application: **27.01.2010**
(21) Application number: 09150772.3
(22) Date of filing: 30.03.2007
(51) Int. Cl.: C07K 14/435, A61K 38/17, A61K 31/00

(54) **Use of 131i-tm-601 for the diagnosis and treatment of gliomas**

(30) Priority: 31.03.2006 US 788145 P; 03.06.2006 US 810279 P
(62) Divisional of application: 07754775.0
(71) Applicant: Transmolecular, Inc., Cambridge, MA 02139 (US)
(72) Inventor: Alvarez, Vernon, L., Morrisville, PA (US)
(74) Representative: Crooks, Elizabeth Caroline

(57) **Abstract**

The present invention is directed to methods and compositions for the - treatment and diagnosis of neuroectodermally-derived tumors, such as gliomas. The inventive methods of treatment generally include local (e.g. intracavitary) administration of the chlorotoxin moiety conjugated to a cytotoxic moiety to a patient. Also provided are diagnostic methods for screening neoplastic neuroectodermal tumors.

## Description

### Related Applications

This application claims priority from Provisional Application U.S.S.N. 60/788,145 filed on March 31, 2006 and Provisional Application U.S.S.N. 60/810,279 filed on June 2, 2006. Each of the above-mentioned provisional applications is incorporated herein by reference in its entirety.

### Background of the Invention

During embryonic development, the future nervous system forms from a specialized layer of ectodermal cells called the neuroectoderm. This layer extends longitudinally along the body axis congruent with the future spinal column. Invagination of the neuroectoderm gives rise to the neural tube from which essentially all central nervous system (CNS) components including the spinal cord develop. Specialized cell clusters along the rim of the invaginating neural tube stay separate from the tube and from the neural crest. These highly migratory neuroectodermal cells give rise to specialized cells throughout the body including Schwann cells, neuronal cells of the peripheral nervous system (PNS) (enteric, parasympathetic, sympathoadrenal, and sensory neurons), pigment calls (melanocytes), endocrine cells and cells forming connective tissue of the face and neck. Since these cells share a common embryonic origin with central nervous system cells, it is not surprising that these cells, or the tumors developing from these cells, share some genetic and antigenic phenotypes with central nervous system cells.

For example, melanomas and glioblastomas share a common mutation in the gene encoding for the epidermal growth factor receptor (EGFR) (M.E. Hegi et al., Int. J. Cancer, 1997, 73: 57-63). Malignant astrocytomas and neurofibromas not only express high levels of the epidermal growth factor receptors but also of the vascular endothelial growth factor receptor (VEGF-R) and platelet-derived growth factor receptor (PDGP-R) (A. Guha, Can J. Neurol. Sci., 1998, 25: 267-281). A high expression of the mutant variant, EGFRvIII, has been demonstrated in glial tumors as well as the extracellular matrix proteins, GP 240 and tenascin (S.N. Kurpad, et al., Glia, 1995, 15: 244-256). Tenascin and the ganglioside-3',6'-isoLD I have been found in both gliomas and primitive neuroectodermal tumor tissues (S.N. Kurpad, et al., Glia, 1995,15: 244-256). In another study, antibodies to tenascin bound extensively to CNS gliomas and also to melanomas, breast, lung and squamous cell carcinomas (D.D. Bigner et al., J. Clin. Onco., 1998, 16: 2202-2212). Another ganglioside, GD2, has been shown to be a common antigen marker in both gliomas and primitive neuroectodermal tumor tissues (D.M. Marcus et al., Invest. Ophthalmol. Vis Sci., 1996, 37: 392-396). Other common antigens between melanomas and gliomas were demonstrated by showing that Tyr, TRP-1, TRP-2 and gp100 gene products are commonly found in both melanoma and glioma tumors (D.D.J. Chi et al., Am. J. Pathol., 1997, 150: 2143-2152). Common cytokines or their receptors linking tumors of astrocytomas, ependymoma, and primitive neuroectodermal tumors have been identified as: interleukin (IL) IL-1 alpha, IL-1, IL-1R1, IL-1R antagonist and transforming growth factor (TGF) TGF-beta 1 (S.E. Ilyin et al., Mol. Chem. Neuropathol., 1998,33:125-137).

Another class of proteins used as markers for gliomas and primitive neuroectodermal tumors are the cytoskeletal proteins, neurofilament (NF), glial fibrillary acidic protein (GFAP), intermediate filaments (IF), intermediate associated protein filament (IFAP), vimentin, nestin and keratins. These markers have been used to determine stages of differentiation along the various cell lineages (R. Kleinert, Acta. Neuropathol., 1991,82: 508-515). New evidence linking astrocytomas with certain primitive neuroectodermal tumors is the cytoskeleton marker of IFAP-300 kDa, a marker of immature glia (H.Y. Yang et al., Mol. Chem. Neuropathol., 1994,21: 155-176).

Further arguments for a tight linkage of neuroectodermally derived cells in the central nervous system and periphery can be made based on their similar dependence on epigenetic influences. For example, sympathoadrenal precursor neurons require basic fibroblast growth factor (bFGF) to proliferate and differentiate, but survival of these cells depends on nerve growth factor (NGF) responsiveness and nerve growth factor availability (E.A. Derrington etal., Oncogene, 1998, 17:1663-1672). A similar scenario is required for each of the other cell types. Not only are growth and trophic factors necessary but cytokines and hormones are needed for which links remain to be elucidated between primitive neuroectodermal tumors and gliomas.

However despite this list of similarities shared between neuroectodermally derived cells, these cells are distinct entities with unique cytological, biochemical and functional features. Indeed, the list of unique features not shared with other neuroectodermally derived cells by far exceeds the above mentioned shared phenotypes. Thus, one can not assume a priori that expression of a certain antigen or phenotype is to be expected in a given cell type based on expression by any other member of the neuroectodermally derived cell types.

Neuroblastomas generally express a selective increase in the gene copy number of the MYCN gene found in fetal stages of brain development suggesting links between the origin of the cells and the ability of neoplastic cells to dedifferentiate (P. Kleihues and K. Webster, Eds. *"Pathology and Genetics of Tumors of the Nervous System. Cavenee",* International Agency for Research on Cancer, Lyon, 1997). However, this gene has yet to be demonstrated in the glioma cells. Other proteins that are not common to both glioma and primitive neuroectodermal tumors have been demonstrated. CD99 immunoreactivity is used as a tool in identifying primitive neuroectodermal tumors (S.Kawauchi et al., Am. J. Surg. Pathol., 1998, 11: 1417-1422) and has been shown in Ewing's sarcoma tumors although not in gliomas (A.A. Renshaw et al., Am. J. Clin. Pathol., 1996, 106: 620-624). Another factor, stem cell factor and its receptor, c-kit, are also expressed in both primitive neuroectodermal tumor and Ewing's Sarcoma tumors (E. Ricotti et al., Blood, 1998,91 : 2397-2405).

The common origin and ability to respond to internal and external signals during the normal developmental processes suggests that central nervous system cells and peripheral neuroectodermally derived cells may also share common mechanisms during pathological developments as for example, during neoplasia. Such neoplastic tissues include CNS gliomas that are glial-derived tumor cells specific to the CNS. They metastasize only within the CNS including the spinal column. They are believed to originate from at least three separate lineages either from undifferentiated precursor cells or by dedifferentiation of astrocytes, oligodendrocytes or ependymal cells.

Primitive neuroectodermal tumors (PNET) are found both in the CNS and PNS. Primitive neuroectodermal tumors found only in the PNS are referred to as peripheral primitive neuroectodermal tumors (PPNET). Primitive neuroectodermal tumors manifest preferentially in children and have capacity for developing into a variety or neuronal, astrocytic, ependymal, muscular and melanotic lines. The conceptual basis of grouping these tumors together is based upon sharing common progenitor cells as well as sharing similar neoplastic transformations leading to tumors of similar morphological features and biological behavior. However, there remains controversy in placing all primitive neuroectodermal tumors into the same category. The following paragraphs demonstrate examples of the overlap of common antigens between the various types of CNS and PNS tumors.

Supratentorial primitive neuroectodermal tumors include cerebral medulloblastomas, cerebral neuroblastomas, "blue" tumors, ependymoblastoma and other primitive neuroectodermal tumors, such as pineoblastomas (WHO grade IV). The most useful markers for these tumors include GFAP, NFP, desmin and melanin. Others antigens found in these tumors are vimentin, nestin, keratin but are not useful for diagnostic purposes.

Peripheral neuroblastic tumors of the adrenal gland (medulla) and sympathetic nervous system are the most common type of childhood tumor outside of the CNS. Primary sites for these primitive neuroectodermal tumors are in the adrenals, abdominal, thoracic, cervical and pelvic sympathetic ganglia but include other primary sites such as orbit, kidney, lung, skin, ovary, spermatic cord, and urinary bladder. Specific names of these related tumors are pheochromocytomas, paraganglioma, neuroblastomas, ganglioneuromas, ganglioneuroblastomas, neurofibromas, schwannomas, and malignant peripheral nerve sheath tumors. These all share common origin in the neural crest. Neuroblastomas all share high TRK-A (NGFR) and CD44 expressions. Neuronal specific enolase (NSE), synaptophysin, neural filament (NF) protein, GD2, tyrosine hydroxylase (TH) and chromogranin are used as diagnostic markers also found in medulloblastomas. Neuroblastomas generally express a selective increase in the gene copy number of the MYCN gene found in fetal stages of brain development (P. Kleihues and K. Webster, Eds. "Pathology and Genetics of Tumors of the Nervous System. Cavenee", International Agency for Research on Cancer, Lyon, 1997).

Medulloblastomas are members of the primitive neuroectodermal tumors that are described as highly malignant embryonal tumors of the CNS found in the cerebellum (WHO grade IV). A common antigen of these medulloblastomas and other neuronal lineage tumors is synaptophysin (not found in glial or mesenchymal brain tumors). Nestin (IF protein) is found in developing CNS precursor cells and in medulloblastomas and in some peripheral neuroectodermal origin cells. Nestin and vimentin are found in medulloblastomas, astrocytomas, glioblastomas, ependymomas, gangliogliomas and meningiomas (only GFAP is found in the astrocytic-derived cells, which are occasionally "trapped" in medulloblastomas). Increased levels of neural-cellular adhesion molecule (N-CAM) found in these tumors, may reflect levels of differentiation in the development of tumors (W.M. Molenaar et al., Acta Neuropathol., 1991,83: 46-54). While varying levels of nerve growth factor (NGF) are found in nearly all tumors, medulloblastomas exhibited substantial reactivity to the NGF receptor and related proteins, neurotrophin (NT) NT-3, TRK-C and brain derived neurotrophic factor (BDNF) (Y. Tajima et al., Acta Neuropathol., 1998, 95: 325-332).

Melanomas, arising from melanocytes follow a graded development from diffuse metanocytosis, to melanocytoma to malignant melanomas. S100 protein is a marker for these tumors, as vimentin and NSE reactivity are variable.

Small cell neuroendocrine carcinomas of the lung are highly invasive and typically found in adult stokers. They have been shown to exhibit reactivity to many of the neural and neuroendocrine markers (some of them similar to N-CAMs) for tumor differentiation as peripheral primitive neuroectodermal tumors, gliomas, and ependymomas. These markers include neural specific enolase and extremely high c-src expression (K. Mellstrom et al., Mol. Cell Biol., 1987, 7: 4178-4184).

A feature conspicuously shared between developing CNS cells and neural crest derived cells is their propensity to migrate either towards a target or target area. It is believed that this ability is lost after cell differentiation and maturation. However, tumors of the CNS show significant cell migration and invasion into healthy brain, suggesting that cells have maintained or regained this enhanced migratory ability. It is, thus, not surprising that neoplastic transformation of neuroectodermally derived cells outside the CNS would have similar migratory abilities. At the intended destination, these cells differentiate into their final phenotype, similar to normal development, influenced by several trophic factors crucial for the proliferation and differentiation of various cell types.

The prior art is deficient in the lack of a diagnostic and therapeutic agents specifically targeted to primitive neuroectodermal tumors.

An estimated 17,000 individuals are diagnosed with glioma annually in the United States. Despite aggressive efforts, the prognosis for survival has not significantly improved in the past 20 years (Adult Brain Tumors (PDQ^{®}): Treatment; National Cancer, http://www.nci.nih.gov/cancertopics/pdq/treatment/adultbrain/health/professional). The five year survival for glioblastoma remains around 3%, and the 2 year survival approximately 8.2% (Accelerate. Brain Cancer Cure (ABC2) website. http://www.abc2.org/statistics.shtml, accessed November 1, 2005). Radiation and chemotherapies seem unlikely to elicit meaningful treatment responses in the near future (L.A. Stewart, Lancet, 2002, 359: 1011-1018; S.A. Grossman and J.F. Batara, Semin. Oncol., 2004, 31: 635-644; LF. Parney and S.M. Chang, Cancer, 2003, 9: 149-156).

Therefore, there is a clear need for alternative strategies for the diagnosis and Treatment of tumors of neuroectodermal origin, such as gliomas.

### Summary of the Invention

The present invention is directed to methods for the diagnosis and treatment of neuroectodermally-derived tumors. More specifically, the present invention encompasses the recognition that TM-601, a synthetic version of a naturally-occurring peptide (chlorotoxin) found in the venom of the Giant Yellow Israeli scorpion *Leiurus Quinquestriatus,* specifically binds to tissue from a wide variety of tumors (see Examples 5-16 and Table 1), suggesting that chlorotoxin-derived molecules can be used to specifically target tumors for therapeutic or diagnostic purposes. In particular, TM-601 was found to specifically bind to tissue from tumors of neuroectodermal origin, both in *vitro* and *in vivo.*

Originally described as a chloride-ion channel blocker, the 36 amino acid chlorotoxin peptide has been explored pre-clinically as a candidate for targeting gliomas with ¹³¹Iodine (J.A. DeBin et al., Am. J. Physiol. (Cell Physiol.), 1993, 264, 33: C361-C369; L. Soroceanu et al., Cancer Res., 1998, 58: 4871-4879; S. Shen et al, Neuro-Oncol., 2005, 71: 113-119). TM-601 is able to cross blood brain and tissue barriers, and may bind to a phosphatidyl inositide, a phosphorylated lipid on lamellipodia of tumor cells, or some yet unidentified receptor (L. Soroceanu et al., Cancer Res., 1998,58: 4871-4879; C.A. Grimes et al., ASCO, 2005 (abstr 9556)). Precinical studies have demonstrated the stability, safety, efficacy, and lack of immunogenicity of radioiodinated TM-601. Based on these data, clinical studies (phase I/II) have been performed to evaluate the safety, tolerability, biodistribution and dosimetry of intracavitary ¹³¹I-TM-601 in adult patients with recurrent high-grade glioma (see Examples 17 and 19). As of February 2007, out of the 18 patients that have received a single dose of ¹³¹I-TM-601 in the Phase I trial, 5 survived 12 months or longer from recurrence; 2 survived more than 36 months from recurrence; and 1 patient remains alive (more than 4 years from recurrence).

Accordingly, in one aspect, the present invention provides methods of treatment of tumors of neuroectodermal origin in a human patient. Such inventive methods of treatment generally involve administration of an effective amount of a chlorotoxin-derived molecule to the patient In certain embodiments, the chlorotoxin-derived molecule comprises at least one chlorotoxin moiety associated with at least one cytotoxic agent Such a molecule is herein called a "cytotoxic chlorotoxin conjugate". The association between the chlorotoxin moiety and cytotoxic moiety may be covalent or non-covalent. In certain embodiments, the chlorotoxin moiety is fused with the cytotoxic moiety to form a fusion protein. The chlorotoxin moiety may be or may be derived from native, synthetic or recombinant chlorotoxin. In certain embodiments, the cytotoxic moiety is selected from the group consisting of toxins, bioactive proteins, and chemotherapeutic agents, nucleolytic enzymes, and radioisotopes. For example, the cytotoxic moiety may comprise gelonin, ricin, saponin, *Pseudomonas* exotoxin, pokeweed antiviral protein, diphtheria toxin, or a complement protein. Alternatively, the cytotoxic moiety may comprise a taxane, a maytansine, a duocarmycin, CC-1065, a auristatin, or a calicheamincin. In certain embodiments, the cytotoxic moiety comprises a radioisotope such as iodine-131 (¹³¹I), iodine-125 (¹²⁵I), technetium-99m (^{99m}Tc), or copper-64 (⁶⁴Cu).

In certain embodiments, the chlorotoxin moiety or the cytotoxic moiety within the conjugate is labeled with a detectable agent, allowing detection and localization of the tumor to be treated.

In certain embodiments, the neuroectodermally-derived tumor to be treated is a member of the group consisting of glioma, meningioma, ependymoma, medulloblastoma, neuroblastoma, ganglioma, pheochromocytoma, melanoma, peripheral primitive neuroectodermal tumor, small cell carcinoma, of the lung, Ewing's sarcoma, and metastatic tumor of neuroectodermal origin in the brain.

In certain embodiments, the neuroectodermally-derived tumor is a refractory tumor.

In certain embodiments, the neumectodermally-defived tumor is located in the brain. In some embodiments, the neuroectodermally-derived tumor is a glioma, for example, a high-grade glioma, particularly a recurrent high-grade glioma.

In certain embodiments where the neuroectodermally-derived tumor is located in the brain, the step of administering the cytotoxic chlorotoxin conjugate comprises intracavitary administration of at least one dose of cytotoxic chlorotoxin conjugate. In some embodiments, the dose of cytotoxic chlorotoxin conjugate comprises between approximately 0.1 mg and approximately 5.0 mg of chlorotoxin moiety. In embodiments where the cytotoxic chlorotoxin conjugate comprises ¹³¹I, the dose of cytotoxic chlorotoxin conjugate may comprise between approximately 10 mCuries and approximately 50 mCuries of ¹³¹I.

In certain embodiments where the neuroectodennally-derived tumor is located in the brain, the step of administering the cytotoxic chlorotoxin conjugate comprises intracavitary administration of at least two doses of cytotoxic chlorotoxin conjugate. For example, 2 doses, 3 doses, 4 doses, 5 doses, 6 doses or more than 6 doses may be administered to a patient. In certain embodiments, two consecutive doses of cytotoxic chlorotoxin conjugate are administered I week apart. In multiple doses methods, each dose of cytotoxic chlorotoxin conjugate may comprise between approximately 0.1 mg and approximately 5.0 mg of chloretoxin moiety. When the cytotoxic chlorotoxin conjugate comprises ¹³¹I, each dose of cytotoxic chlorotoxin conjugate may comprise between approximately 10 mCuries and approximately 50 mCuries of ¹³¹I.

In certain embodiments of the methods of treatment of the present invention, the cytotoxic chlorotoxin conjugate is administered in combination with a chemotherapeutic agent

In another aspect, the present invention provides methods for the diagnosis of neuroectodermally-derived tumors. More specifically, methods are provided for differentiating netroectudermally-derived neoplastic tumor tissue from non-neoplastic tissue. Generally, such methods comprise steps of: contacting a tissue of interest with a diagnostic chlorotoxin agent, wherein the diagnostic chlorotoxin agent comprises at least one chlorotoxin moiety associated with at least one labeling moiety; and measuring binding of the diagnostic chlorotoxin agent to the tissue of interest, wherein an elevated level of binding, relative to normal tissue, indicates that the tissue is a neuroectodermally-derived tumor tissue.

The labeling moiety facilitates visualization of the diagnostic agent that binds to the tissue of interest. Examples of labeling moieties include, but are not limited to, fluorescent agents, radioisotopes, enzymes, paramagnetic metal ions, and the like. Detection of the binding may be performed using any of wide-variety of methods such as, for example, fluorescence microscopy, bioluminescence, fluorescent activated cell sorting, histochemical staining, ELISA, Magnetic Resonance Imaging (MRI), Gamma camera, Single Photon Emission Computed Tomography (SPECT) or Positron Emission Tomography (PET).

In certain embodiments, the contacting step is performed *in vivo* (*i.e.,* in a individual suspected of having a tumor of neuroectodermal origin. In other embodiments, the contacting is performed *in vitro* or *ex vivo* (*e.g*., using a tissue sample obtained from an individual suspected of having a tumor of neuroectodermal origin).

In certain preferred embodiments, methods provided by the present invention are used for the diagnosis of a glíoma meningioma, ependymoma, medulloblastoma, neuroblastoma, ganglioma, pheochromocytoma, melanoma, peripheral primitive neuroectodermal tumor, small cell carcinoma of the lung, Ewing's sarcoma, or a metastatic tumor of neuroectodermal origin in the brain.

### Description of the Drawing

FIG. 1 shows the positive immunohistochemical staining of a glioblastoma multiform (GBM) tumor with chlorotoxin. The brown reaction product of DAB 3'3'-diaminobensdine with biotinylated chlorotoxin is clearly visible in the TM-601 stained section. TM-601: biotinylated chlorotoxin stained, counter-stained with methyl green; Control: methyl green only; and, H&E stain: hematoxylin and eosin.

FIG. 2 demonstrates that nornal brain is not immunohistochemically stained by biotinylated chlorotoxin. TM-601: biotinylated chlorotoxin, counter-stained with methyl green; Control: methyl green only; and, H&E stain: hematoxylin and eosin. Normal brain was also stained with biotinylated antibodies against GFAP (Glial Fibrillary Acidic Protein) which positively stains astrocytes in normal brain tissue.

FIG. 3 shows chlorotoxin staining of an adrenal mass neuroblastoma tumor. TM-601: biotinylated chlorotoxin, counter-stained with methyl green; Control: methyl, green only; and, H&E stain: hematoxylin and eosin.

FIG. 4 illustrates that biotinylated chlororoxin immunohistochemically stains pheochromocytomas. TM-601: biotinylated chlorotoxin, counter-stained with methyl green; Control: methyl green only; and, H&E stain: hematoxylin and eosin.

FIG. 5 illustrates that normal adrenal tissue is not immohistochemically stained by biotinylated chlorotoxin. TM-601: biotinylated chlorotoxin, counter-strained with methyl green; Control: methyl green only; and, H&E stain: hematoxylin and eosin.

FIG. 6 shows the immunohistochemical staining with biotinylated chlorotoxin of melanoma tumor cells metastasized to the brain. TM-601: biotinylated chlorotoxin, counter-stained with methyl green; Control: methyl green only; and, H&E stain: hematoxylin and eosin.

FIG. 7 illustrates biotinylated chlorotoxin immunohistochemical staining of melanoma tumor cells metastasized to the lung. TM-601: biotinylated chlorotoxin, counter-stained with methyl green; Control: methyl green only; and, H&E stain: hematoxylin and eosin.

FIG. 8 shows immunohistochemical staining of normal skin with biotinylated chlorotoxin. TM-601: biotinylated chlorotoxin, counter-stained with methyl green; Control: methyl green only; and, H&E stain: hematoxylin and eosin.

FIG. 9 shows immunohistochemical staining of small cell lung carcinomas with biotinylated chlorotoxin. TM-601: biotinylated chlorotoxin, couster-stained with methyl green; Control: methyl green only; and, H&E stain: hematoxylin and eosin.

FIG. 10 shows that biotinylated chlorotoxin does not immunohistochemically stain normal lung tissue. TM-601: biotinylated chlorotoxin, counter-stained with methyl green; Control: methyl green only; and, H&E stain: hematoxylin and eosin.

FIG. 11 shows the immunohistochemical staining of a medulloblastoma tumor with biotinylated chlorotoxin. TM-601: biotinylated chlorotoxin, counter-stained with methyl green; Control: methyl green only; and, H&E stain: hematoxylin and eosin.

FIG. 12 shows that the rare, neuroectodermally derived bone cancer, Ewing's sarcoma, also exhibits positive immunohistochemical staining with biotinylated chlorotoxin. TM-601: biotinylated chlorortoxin, counter-stained with methyl green; Control: methyl green only; and, H&E stain: hematoxylin and eosin.

FIG. 13 shows the negative results obtained in the immunohistochemical staining of normal stomach tissue with biotinylated chlorotoxin. TM-601: biotinylated chlorotoxin, counter-stained with methyl green; Control: methyl green only; and, H&E stain: hematoxylin and eosin.

FIG. 14 shows the lack of immunohistochemical staining of normal liver tissue with biotinylated chlorotoxin. TM-601: biotinylated chlorotaxin, counter-stained with methyl green; Control: methyl green only; and, H&E stain: hematoxylin and eosin.

FIG. 15 demonstrated that normal spleen tissue is not immunohistochemically stained by biotinylated chlorotoxin. TM-601: biotinylated chlorotoxin, counter-stained with methyl green; Control: methyl green only; and, H&E stain: hematoxylin and eosin.

FIG. 16 depicts length of survival for each patient (as of November 2005) following treatment with 10 mCi ¹³¹I-TM-601 measured in weeks.

FIG. 17 depicts results of anterior gamma camera scans for patient 104. Intense localization is seen in the surgical cavity as late as 5 days post infusion. This result was confirmed on brain SPECT scans and was observed uniformly in all patients. Note the location of hot-spots outside the surgical cavity (arrows).

FIG. 18 illustrates representative histology of a GBM patient sample (patient 0204). Frozen sections were fixed and stained with biotinylated peptide. Streptavidin-HRP and DAB were used for detection as indicated by the intense brown staining with biotinylated TM-601 in panel A. **A**. 10 µM biotinylated TM-601. B. No peptide negative control. **C**. H&E. Radiographic response of this patient is shown in panels **D-F,** indicating stable disease at 180 days post treatment.

FIG. 19 depicts length of survival for each patient (as of April 2006) following treatment with ¹³¹I-TM-601 described in Example 18 (survival measured in months).

FIG. 20 depicts length of survival for each patient (as of February 2007) following treatment with 10 mCi ¹³¹I-TM-601 measured in weeks (see Example 17).

FIG. 21 shows the survival of patients treated as described in Examples 17, compared to the survival of a group of patients with recurrent glioblastoma multiforme treated with Gliadel^{®} wafer (H. Brem et al , Lancet, 1995, 345: 1008-1012).

FIG. 22 depicts length of survival for each patient (as of February 2007) following Treatment with ¹³¹I-TM-601 described in Example 18 (survival measured in months).

TABLE I shows a summary of the results of staining of various human tissue with TM-601

FIG. 23 demonstrates that chlorotoxin labeled with ^{99m}Tc binds to human tumor cells (U87, A459, and PC3), but not to normal cells (NHDF = normal human dermal fibroblasts).

### Definitions

Throughout the specification, several terms are employed that are defined in the following paragraphs.

The terms ***"individual", "subject"*** and ***"patient"*** are used herein interchangeably. They refer to a human being that can be afflicted with a tumor of neuroectodermal origin but may or may not have such a tumor. The terms do not denote a particular age, and thus encompass adults, teenagers, and children.

The term ***"treatment"*** is used herein to characterize a method or process that is aimed at (1) delaying or preventing the onset of a disease or condition; (2) slowing down or stopping the progression, aggravation, or deterioration of the symptoms of the disease or condition; (3) bringing about ameliorations of the symptoms of the disease or condition; or (4) curing the disease or condition. A treatment may be administered prior to the onset of the disease, for a prophylactic or preventive action. Alternatively or additionally, the treatment may be administered after initiation of the disease or condition, for a therapeutic action.

As used herein, the term ***"effective amount"*** refers to any amount of a compound or composition that is sufficient to fulfill its intended purpose(s), *i.e.,* a desired biological or medicinal response in a tissue or subject. For example, in certain embodiments of the present invention, the purpose(s) may be: to specifically bind to a target tissue, to slow down or stop the progression, aggravation, or deterioration of the symptoms of a neuroectodermally-derived cancer, to bring about amelioration of the symptoms of the cancer, and/or to cure the cancer.

A ***"pharmaceutical compositions"*** is herein defined herein as comprising an effective amount of at least one active ingredient (*e.g.,* a cytotoxic chlorotoxin moiety), and at least one pharmaceutically acceptable carrier.

As used herein, the term ***"pharmaceutically acceptable carrier"*** refers to a carrier medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, absorption delaying agents, and the like. The use or such media and agents for pharmaceutically active substances is well known in the art (see for example ***"Remington's Pharmaceutical Sciences",*** E.W. Martin, 18^{th} Ed., 1990, Mack Publishing Co.: Easton, PA, which is incorporated herein by reference in its entirety).

The terms ***"approximately"*** and ***"about",*** as used herein in reference to a number generally includes numbers that fall within a range of 10% in either direction of the number (greater than or less than the number) unless otherwise stated or otherwise evident from the context (except where such number would exceed 100% of a possible value).

The term ***"cytotoxic",*** when used herein to characterize a moiety, a compound, a drug or an agent refers to a moiety, a compound, a drug or an agent that inhibits or prevents the function of cells and/or causes destruction of cells.

The terms ***"therapeutic agent"*** and ***"drug"*** are used herein interchangeably. They refer to a substance, molecule, compound, agent, factor or composition effective in the treatment of a disease or clinical condition.

The terms ***"chemotherapeutics"*** and ***"anti-cancer agents*** or ***drugs"*** are used herein interchangeably. They refer to those medications that are used to treat cancer or cancerous conditions. Anti-cancer drugs are conventionally classified in one of the following group: alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifies, anti-hormones and anti-androgens. Examples of such anti-cancer agents include, but are not limited to, BCNU, cisplatin, gemcitabine, hydroxyurea, paclitaxel, temozomide, topotecan, fluorouracil vincristine, vinblastine, procarbasine, decarbazine, altretamine, methotrexate, mercaptopurine, thioguanine, fludarabine phosphate, cladribine, pentostatin, cytarabine, azacitidine, etoposide, teniposide, irinotecan, docetaxel doxorubicin, daunorubicin, dactinomycin, idarubicin, plicamycin mitomycin, bleomysin tamoxifen, flutamide, leuprofide, goserelin, aminogluthimide, anastrozole, amsacrine, asparaginase, mitoxantrone, mitotane and amifostine.

The term ***"small molecule"*** includes any chemical or other moiety that can act to affect biological processes. Small molecules can include any number of therapeutic agents presently known and used, or can be small molecules synthesized in a library of such molecules for the purpose of screening for biological function(s). Small molecules are distinguished from macromolecules by size. Small molecules suitable for use in the present invention usually have molecular weight less than about 5,000 daltons (Da), preferably less than about 2,500 Da, more preferably less than 1,000 Da, most preferably less than about 500 Da.

The terms ***"protein", "polypeptide",*** and ***"peptide"*** are used herein interchangeably, and refer to amino acid sequences of a variety of lengths, either in their neutral (unchanged) forms or as salts, and either unmodified or modified by glycosylation, side chain oxidation, or phosphorylation. In certain embodiments, the amino acid sequence is the full-length native protein. In other embodiments, the amino acid sequence is a smaller fragment of the full-length protein. In still other embodiments, the amino acid sequence is modified by additional substituents attached to the amino acid side chains, such as glycosyl units, lipids, or inorganic ions such as phosphates, as well as modification relating to chemical conversion of the chains, such as oxidation of sulfhydryl groups. Thus, the term "protein" (or its equivalent terms) is intended to include the amino acid sequence of the full-length native protein, subject to those modification that do not change its specific properties. In particular, the term "protein" encompasses protein isoforms, *i.e.,* variants that are encoded by the same gene, but that differ in their pI or MW, or both. Such isoforms can differ in their amino acid sequence (*e.g*., as a result of alternative slicing or limited proteolysis), or in the alternative, may arise from differential post-translational modification (*e.g*., glycosylation, acylation or phosphorylation).

The term **"*protein analog*",** as used herein, refers to a polypeptide that possesses a similar or identical function as the full-length native protein but need not necessarily comprise an amino acid sequence that is similar or identical to the amino acid sequence of the protein, or possesses a structure that is similar or identical to that of the protein. Preferably, in the context of the present invention, a protein analog has an amino acid sequence that is at least 30% (more preferably, at least 35%, at least 40%, at least 45%, at least 50%, at least 55%, at least 60%, at least 65%, at least 70%, at least 75%, at least 80%, at least 85%, at least 90%, at least 95% or at least 99%) identical to the amino acid sequence of the full-length native protein.

The term **"*protein fragment*",** as used herein, refers to a polypeptide comprising an amino acid sequence of at least 5 amino acid residues of the amino acid sequence of a second polypeptide. The fragment of a protein may or may not possess a functional activity of the full-length native protein.

The term **"*biologically active*",** when used herein to characterize a protein variant, analog or fragment, refers to a molecule that shares sufficient amino acid sequence homology with the protein to exhibit similar or identical properties than the protein (*e.g*., ability to specifically bind to cancer cells and/or to be internalized into cancer cells and/or to kill cancer cells).

The term **"*homologous*"** (or **"*homologous*"**), as used herein, is synonymous with the term **"*identity*"** and refers to the sequence similarity between two polypeptides, molecules or between two nucleic acid molecules. When a position in both compared sequences is occupied by the same base or amino acid monomer subunit, then the respective molecules are homologous at that position. The percentage of homology between two sequences corresponds to the number of matching or homologous positions shared by the two sequences divided by the number of positions compared and multiplied by 100. Generally, a comparison is made when two sequences are aligned to give maximum horology. Homologous amino acid sequences share identical or similar amino acid residues. Similar residues are conservative substitutions for, or "allowed point mutations" of, corresponding amino acid residues in a reference sequence. "Conservative substitutions" of a residue in a reference sequence are substitutions that are physically or functionally similar to the corresponding reference residue, *e.g.,* that have a similar size, shape, electric charge, chemical properties, including the ability to form covalent or hydrogen bonds, or the like. Particularly preferred conservative substitutions are those fulfilling the criteria defined for an "accepted point mutation" by Dayhoff et al. ("Atlas of Protein Sequence and Structure", 1978, Nat. Biomed. Res. Foundation, Washington, DC, Suppl. 3, 22: 354-352).

The term **"*fusion protein*"** refers to a molecule comprising two or more proteins or fragments thereof linked by a covalent bond via their individual peptide backbones, most preferably generated through genetic expression of a polynucleotide molecule encoding those proteins.

The terms **"*normal*"** and **"*healthy*"** are used herein interchangeably. They refer to an individual or group of individuals who do not have a tumor of neuroectodermal origin. In certain embodiments of diagnostic methods of the present invention, normal individuals have similar sex, age, and/or body mass index as compared with the individual to be diagnosed. The term "normal" is also used herein to qualify a tissue sample isolated from a healthy individual.

The term "***tissue***" is used herein in its broadest sense. A tissue may, be any biological entity that can (but may not necessarily) comprise a tumor cell of neuroectodermal origin. In the context of the present invention, *in vitro, in vivo* and ex vivo tissues are considered. Thus, a tissue may be part of an individual or may be obtained from an individual (*e.g.*, by biopsy). Tissues may also include sections of tissue such as frozen sections taken for histological purposes or archival samples with knows diagnosis, treatment and/or outcome history. The term tissue also encompasses any material derived by processing the tissue sample. Derived materials include, but are not limited to, cells (or their progeny) isolated from the tissue. Processing of the tissue sample may involve one or more of filtration, distillation, extraction, concentration, inactivation of interfering components, addition of reagents, and the like.

The terms **"*labeled*"** and **"*labeled with a detectable agent*** or ***moiety*"** are used herein interchangeably to specify that an entity (*e.g*., a chlorotoxin or chlorotoxin derivative) can be visualized, for example following binding to another entity (*e.g.*, a neoplastic tumor tissue of neuroectodermal origin). Preferably the detectable agent or moiety is selected such that it generates a signal which can be measured and whose intensity is related to (*e.g.*, proportional to) the amount of bound entity. A wild variety of systems for labeling and/or detecting proteins and peptides are known in the art. Labeled proteins and peptides can be prepared by incorporation of, or conjugation to, a label that is detectable by spectroscopic, photochemical, biochemical, immunochemical, electrical, optical, chemical or other means. A label or labeling moiety may be directly detectable (*i.e*., it does not require any further reaction or manipulation to be detectable, *e.g*., a fluorophore is directly detectable) or it may be indirectly detectable (*i.e*., it is made detectable through reaction or binding with another entity that is detectable, *e.g*., a hapten is detectable by immunostaining after reaction with an appropriate antibody comprising a reporter such as a fluorophore). Suitable detectable agents include, but are not limited to, radionuclides, fluorophores, chemiluminescent agents, microparticles, enzymes, colorimetric labels, magnetic labels; haptens, Molecular Beacons, aptamer beacons, and the like.

### Detailed Description of Certain Preferred Embodiments

As already mentioned above, the present invention is directed to methods for the treatment and diagnosis of tumors of neuroectodermal origin in humans. The methods provided by the present invention are, for example, useful in the treatment and diagnosis of brain tumors of neuroectodermal origin, such as gliomas, in particular high-grade gliomas.

In accordance with the present invention there may be employed conventional molecular biology, microbiology, and recombinant DNA techniques within the skill of the art. Such techniques are explained fully in the literature. See, *e.g.*, Maniatis, Fritsch & Sambrook, "Molecular Cloning: A Laboratory Manual", 1982; "DNA Cloning: A Practical Approach," Volumes I and II, D.N. Glover (Ed.), 1985; "Oligonucleotide Synthesis", M.J. Gait (Ed.), 1984; "Nucleic Acid Hybridization", B.D. Hames & S.J. Higgins (Eds.), 1985; "Transcription and Translation" B.D. Hames & S.J, Higgins(Eds.),1984; "Animal Cell Culture", R.I. Freshney (Ed.), 1986; "Immobilized Cells And Enzymes". IRL Press, 1986; B. Perbal, "A Practical Guide To Molecular Cloning", 1984.

### I. Cytotoxic Chlorotoxin Conjugates

Methods of treatment of the present invention involve administering, to an individual in need thereof, an effective amount of a cytotoxic chlorotoxin conjugate. A conjugate generally is a molecule resulting from the binding, interaction, association or coupling of at least two other molecules. As used herein, the term "a cytotoxic chlorotoxin conjugate" refers to a molecule comprising at least one chlorotoxin moiety associated with at least one cytotoxic moiety.

The interaction between a chlorotoxin moiety and a cytotoxic agent within a conjugate may be covalent or non-covalent. Irrespective of the nature of the interaction, the binding, association, or coupling between the chlorotoxin moiety and cytotoxic moiety is preferably selective, specific and strong enough so that the conjugate does not dissociate before or during transport/delivery of the conjugate to the tumor. Association between the chlorotoxin moiety and cytotoxic moiety may be achieved using any chemical, biochemical enzymatic, or genetic coupling known to one skilled in the art.

In certain embodiments, the interaction between the chlorotoxin moiety and cytotoxic moiety is non-covalent. Examples of non-covalent interactions include, but are not limited to, hydrophobic interactions, electrostatic interactions, dipole interactions, van der Waals interactions, and hydrogen bonding.

In certain embodiments, the cytotoxic moiety is covalently attached to the chlorotoxin moiety. As will be appreciated by one skilled in the art, the cytotoxic agent and chlorotoxin moiety may be attached to each other either directly or indirectly (*e.g*., through a linker).

In certain embodiments, the cytotoxic moiety and chlorotoxin moiety are directly covalently linked to each other. The direct covalent binding can be through a linkage such as an amide, ester, carbon-carbon, disulfide, carbamate, ether, thioether, urea, amine, or carbonate linkage. The covalent binding can be achieved by taking advantage of functional groups present on the cytotoxic agent and/or the chlorotoxin moiety. Alternatively, a non-critical amino acid may be replaced by another amino acid which will introduce a useful group (amino, carboxy or sulfhydryl) for coupling purposes. Alternatively, an additional amino acid may be added to the chlorotoxin moiety to introduce a useful group (amino, carboxy or sulfhydryl) for coupling purposes. Suitably functional groups that can be used to attach two moieties together include, but are not limited to, amines, anhydrides, hydoxyl groups, carboxy groups, thiols, and the like. An activating agent, such as a carbodiimide, can be used to form a direct linkage. A wide variety of activating agents are known in the art and are suitable for linking a cytotoxic agent and a chlorotoxin moiety.

In other embodiments, the cytotoxic agent and chlorotoxin moiety are indirectly covalently linked to each other via a linker group. This can be accomplished by using any number of stable bifunctional agents well known in the art, including homofunctional and heterofunctional agents (for examples of such agents, see, *e.g.*, Pierce Catalog and Handbook). The use of a bifunctional linker differs from the use of an activating agent in that the former results in a linking moiety being present in the resulting conjugate, whereas the latter results in a direct coupling between the two moieties involved in the reaction. The role of a bifunctional linker may be to allow reaction between two otherwise inert moieties. Alternatively or additionally, the bifunctional linker, which becomes part of the reaction produce may be selected such that it confers some degree of conformational flexibility to the conjugate (*e.g*., the bifunctional linker comprises a straight alkyl chain containing several atoms, for example, the straight alkyl chain contains between 2 and 10 carbon atoms). Alternatively, or additionally, the bifunctional linker may be selected such that the linkage formed between the cytotoxic agent and the chlorotoxin moiety is cleavable, *e.g.* hydrolysable (for examples of such linkers, see *e.g*. U.S. Pat. Nos. 5,773,001; 5,739,116 and 5,877,296, each of which is incorporated herein by reference in its entirety). Such linkers are for example preferably used when higher activity of the chlorotoxin moiety and/or of the cytotoxic moiety is observed after hydrolysis of the conjugate. Exemplary mechanisms by which a cytotoxic moiety may be cleaved from a chlorotoxin moiety include hydrolysis in the acidic pH of the lysosomes (hydrazones, acetals, and cis-aconitate-like amides), peptide cleavage by lysosomal enzymes (the capthepsins and other lysosomal enzymes), and reduction of disulfides).

For example, hydrazone-containing conjugates can be made with introduced carbonyl groups that provide the desired release properties. Conjugates can also be made with a linker that comprise an alkyl chain with a disulfide group at one end and a hydrazine derivative at the other end. Linkers containing functional groups other than hydrazones also have the potential to be cleaved in the acidic milieu of lysosomes. For example, conjugates can be made from thiol-reactive linkers that contain a group other than a hydrazone that is cleavable intracellularly, such as esters, amides, and acetals/ketals.

Another example of class of pH sensitive linkers are the cis-aconitates, which have a carboxylic acid group juxtaposed to an amide group. The carboxylic acid accelerates amide hydrolysis in the acidic lysosomes. Linkers that achieve a similar type of hydrolysis rate acceleration with several other types of structures can also be used.

Another potential release method for cytotoxic agent-chlorotoxin conjugates is the enzymatic hydrolysis of peptides by the lysosomal enzymes. In one example, a peptidic toxin is attached via an amide bond to para-aminobenzyl alcohol and then a carbamate or carbonate is made between the benzyl alcohol and the cytotoxic agent Cleavage of the peptide leads to collapse of the amino benzyl carbamate or carbonate, and release of the cytotoxic agent. In another example, a phenol can be cleaved by collapse of the linker instead of the carbamate. In another variation, disulfide reduction is used to initiate the collapse of a para-mercaptobenzyl carbamate or carbonate.

In embodiments where the cytotoxic agent is a protein, a polypeptide or a peptide, the cytotoxic chlorotoxin conjugate may be a fusion protein. As already defined above, a fusion protein is a molecule comprising two or more proteins or peptides linked by a covalent bond via their individual peptide backbones. Fusion proteins used in methods of the present invention can be produced by any suitable method known in the art. For example, they can be produced by direct protein synthetic methods using a polypeptide synthesizer. Alternatively, PCR amplification of gene fragments can be carried out using anchor primers which give rise to complementary overhangs between two consecutive gene fragments that can subsequently be annealed and re-amplified to generate a chimeric gene sequence. Fusion proteins can be obtained by standard recombinant methods (see, for example, Maniatis et al. "Molecular Cloning: A Laboratory Manual". 2nd Ed., 1989, Cold Spring Harbor Laboratory, Cold Spring, N.Y.). These methods generally comprise (1) construction of a nucleic acid molecule that encodes the desired fusion protein; (2) insertion of the nucleic acid molecule into a recombinant expression vector; (3) transformation of a suitable host cell with the expression vector; and (4) expression of the fusion protein in the host cell. Fusion proteins produced by such methods may be recovered and isolated, either directly from the culture medium or by lysis of the cells, as known in the art. Many methods for purifying proteins produced by transformed host cells are well-known in the art. These include, but are not limited to, precipitation, centrifugation, gel filtration, and (ionexchange, reverse-phase, and affinity) column chromatography. Other purification methods have been described (see, for example, Deutscher et al. "Guide to Protein Purification" in Methods in Enzymology, 1990, Vol. 182, Academic Press).

As can readily be appreciated by one skilled in the art, a cytotoxic chlorotoxin conjugate used in methods of the present invention can comprise any number of chlorotoxin moieties and any number of cytotoxic moieties, associated to one another by any number of different ways. The design of a conjugate will be influenced by its intended purpose(s) and the properties that are desirable in the particular context of its use. Selection of a method to associate or bind a chlorotoxin moiety to a cytotoxic agent to form a conjugate is within the knowledge of one skilled in the art and will generally depend on the nature of the interaction desired between the moieties (*i.e*., covalent vs. non-covalent and/or cleavable *vs*. non-cleavable), the nature of the cytotoxic agent, the presence and nature of functional chemical groups on the moieties involved and the like.

### A. Chlorotoxin Moieties

Conjugates used in methods of treatment of the present invention comprise at least one chlorotoxin moiety. As used herein, the term "***chlorotoxin moiety***" refers to a chlorotoxin, a biologically active subunit thereof or a biologically active derivative thereof

In certain embodiments, the term "*chlorotoxin*" refers to the full-length, 36 amino acid polypeptide naturally derived from *Leiurus quinquestritus* scorpion venom (DeBin *et al*., Arm. J. Physiol., 1993, 264: C361-369), which comprises the amino acid sequence of native chlorotoxin as set forth in SEQ ID NO. 1 of International Application No. WO 2003/101474, the contents of which are incorporated herein by reference. The term "chlorotoxin" includes polypeptides comprising SEQ ID NO. 1 which have been synthetically or recombinantly produced, such as those disclosed in U.S. Pat. No. 6,319,891 (which is incorporated herein by reference in its entirety).

A "***biologically active chlorotoxin subunit*"** is a peptide comprising less than the 36 amino acids of chlorotoxin and which retains at least one property or function of chlorotoxin. As used herein, a "property or function" of chlorotoxin includes, but is not limited to, the ability to arrest abnormal cell growth, ability to specifically bind to a tumor/cancer cell compared to a normal cell, ability to be internalized into a tumor/cancer cell, and ability to kill a tumor/cancer cell. The tumor/cancer cell may be *in vitro, ex vivo*, *in vitro*, a primary isolate from a subject, a cultured cell, or a cell line.

As used herein, the term *"biologically active chlorotoxin derivative"* refers to any of a wide variety- of- derivatives, analogs, variants, polypeptides fragment and mimetics of chlorotoxin and related peptides which retain at least one property or function of chlorotoxin (as described above). Examples of chlorotoxin derivatives include, but are not limited to, peptide variants of chlorotoxin, peptide fragments of chlorotoxin, for example, fragments comprising or consisting of contiguous 10-mer peptides or SEQ ID No. 1, 2, 3, 4, 5, 6, or 7 as set forth in International Application No. WO 2003/101474 or comprising residues 10-18 or 21-30 or SEQ ID No. 1 as set forth in International Application No. WO 2003/101474, core binding sequences, and peptide mimetics.

Examples of chlorotoxin derivatives include peptides having a fragment of the amino acid sequences set forth in SEQ ID No. 1 of International Application No. WO 2003/101474, having at least about 7,8, 9, 10,15,20,25, 30 or 35 contiguous amino acid residues, associated with the activity of chlorotoxin. Such fragments may contain functional regions of the chlorotoxin peptide, identified as regions of the amino acid sequence which correspond to known peptide domains, as well as regions of pronounced hydrophilicity. Such fragments may also include two core sequences linked to one another, in any order, with intervening amino acid removed or replaced by a linker.

Derivatives of chlorotoxin include polypeptide comprising a conservative or non-conservative substitution of at least one amino acid residue when the derivative sequence and the chlorotoxin sequence are maximally aligned. The substitution may be one which enhances at least one property or function of chlorotoxin, inhibits at least one property or function of chlorotoxin, or is neutral to at least one property or function of chlorotoxin.

Examples of derivatives of chlorotoxin suitable for use in the practice of the present invention are described in International Application No. WO 2003/101474 (which is incorporated herein by reference in its entirety). Particular examples include polypeptides that comprise or consist of SEQ ID NO. 8 or SEQ ID NO. 13 as set forth in this International Application, as well as variants, analogs, and derivatives thereof.

Other examples of chlorotoxin derivatives include those polypeptides containing pre-determined mutations by, *e.g*., homologous recombination, site-directed or PCR mutagenesis, and the alleles or other naturally-occurring variants of the family of peptides; and derivatives wherein the peptide has been covalently modified by substitution, chemical, enzymatic or other appropriate means with a moiety other than a naturally-occurring amino acid (for example a detectable moiety such as enzyme or a radioisotope).

Chlorotoxin and peptide derivatives thereof can be prepared using any of a wide variety of methods, including standard solid phase (or solution phase) peptide synthesis methods, as is known in the art. In addition, the nucleic acids encoding these peptides may be synthesized using commercially available oligonucleotide synthesis instrumentation and the proteins may be produced recombinantly using standard recombinant production systems.

Other suitable chlorotoxin derivatives include peptide mimetics that mimic the three-dimensional structure of chlorotoxin. Such peptide mimetícs may have significant advantages over naturally occurring peptides including, for example, more economical production, greater chemical stability, enhanced pharmacological properties (half-life, absorption, potency, efficacy, etc), altered specificity (*e.g*., broad-spectrum biological activities, reduced antigenicity and others).

In certain embodiments, mimetics are molecules that mimic elements of chlorotoxin peptide secondary structure. Peptide backbone of proteins exists mainly to orient amino acid side chains in such a way as to facilitate molecular interactions, such as those of antibody and antigen. A peptide mimetic is expected to permit molecular interactions similar to the natural molecule. Peptide analogs are commonly used in the pharmaceutical industry as non-peptide drugs with properties analogous to those of the template peptide. These types of compounds are also referred to as peptide mimetics or peptidomimetics (see, for examples, Fauchere, Adv. Drug Res., 1986, 15: 29-69; Veber & Freidinger, 1985, Trends Neurosci., 1985, 8: 392-396; Evans et al., J. Med. Chem., 1987, 30: 1229-1239) and are usually developed with the aid of computerized molecular modeling.

Generally, peptide mimetics are structurally similar to a paradigm polypeptide (*i.e.,* a polypeptide that has a biochemical property or pharmacological activity), but have one or more peptide linkages optionally replaced by a non-peptide linkage. The use of peptide mimetics can be enhanced through the use of combinatorial chemistry to create drug libraries. The design of peptide mimetics can be aided by identifying amino acid mutations that increase or decrease the binding of a peptide to, for example, a tumor cell. Approaches that can be used include the yeast two hybrid method (see, for example, Chien et al., Proc. Natl. Acad. Sci. USA, 1991, 88: 9578-9582) and using the phase display method. The two hybrid method detects protein-protein interactions in yeast (Field et al., Nature, 1989, 340: 245-246). The phage display method detects the interaction between an immobilized protein and a protein that is expressed on the surface of phages such as lambda and M13 (Amberg et al., Strategies. 1993, 6: 2-4; Hogrefe et al, Gene, 1993, 128: 119-126). These methods allow positive and negative selection of peptide-protein interaction and the identification of the sequences that determine these interactions.

In certain embodiments, the term "chlorotoxin" refers to polypeptide toxins of other scorpion species that display similar or related activity to chlorotoxin described above. As used herein, the term "similar or related activity to chlorotoxin" refers, in particular, to the selective/specific binding to tumor/cancer cells. Examples of suitable related scorpion toxins include, but are not limited to toxins or related peptides of scorpion origin, that display amino acid and/or nucleotide sequence identity to chlorotoxin. Examples of related scorpion toxins include, but are not limited to, CT neurotoxin from *Mesobuthus* martenssi (GenBank Accession No.AAD473730), Neurotoxin BmK 41-2 from *Buthus martensii karsch* (GenBank Accession No. A59356), Neurotoxin Bm12-b from *Buthus martensii* (GenBank Accession No. AAK16444), Probable Toxin LGH 8/6 from *Leiurus quinquestriatus hebraeu* (GenBank Accession No. P55966), Small toxin from *Mesubutus tamulus sindicus* (GenBank Accession No. P15229).

Related scorpion toxins suitable for use in the present invention comprise polypeptides that have an amino acid sequence of at least about 75%, at least about 85%, at least about 0%, at least about 95%, or at least about 99% sequence identity with the entire chlorotoxin sequence as set forth in SEQ ID No. 1 of International Application No. WO 2003/101474 (which is incorporated herein by reference in its entirety). In certain embodiments, related scorpion toxins include those scorpion toxins that have a sequence homologous to SEQ ID NO. 8 or SEQ ID NO. 13 of chlorotoxin, as set forth in International Application No. WO 2003/101474.

In certain embodiments, the chlorotoxin moiety within a conjugate is labeled. In other embodiments, the cytotoxic moiety within a conjugate is labeled. Examples of labeling methods and labeling agents are described below.

### B. Cytotoxic Agents

In cytotoxic chlorotoxin conjugates used in methods of treatment of the present invention, a chlorotoxin moiety is associated with a cytotoxic agent (or moiety). Suitable cytotoxic agents include any of a large variety of substances, molecules compounds, agents or factors that are toxic to living cells.

As will be appreciated by one or ordinary skill in the art, a cytotoxic agent may be a synthetic or natural compound; a single molecule, a mixture of different molecules or a complex of different molecules. Suitable cytotoxic agents can belong to any of various classes of compounds including, but not limited to, small molecules, peptide, proteins, saccharides, steroids, antibodies (including fragments and variants thereof), fusion proteins, antisense polynucleotides, ribozymes, small interfering RNAs, peptidomimetics, radionuclides, and the like. When cytotoxic agents are used in the treatment of cancer or a cancerous condition they can be found, for example, among the following classes of anti-cancer drugs: alkylating gents, anti-metabolic drugs, anti-mitotic antibiotics, alkaloidal anti-tumor agents, hormones and anti-hormones, interferons, non-steroidal anti-inflammatory drugs, and various other anti-tumor agents such as kinase inhibitors, proteasome inhibitors and NF-κB inhibitors.

Examples of suitable cytotoxic agents for use in cytotoxic chlorotoxin conjugates include toxins, other bioactive proteins, conventional chemotherapeutic agents, enzymes, and radioisotopes.

Examples of suitable toxins include, but are not limited to, bacterial and plant toxins such as gelonin, ricin, saponin, *Pseudomonas* exotoxin, pokeweed antiviral protein, and diphtheria toxin.

Examples of suitable bioactive proteins includes, but are not limited to, proteins of the complement system (or complement proteins). The complement system is a complex biochemical cascade that helps clear pathogens from an organism, and promote healing (B.P. Morgan, Crit. Rev. Clin. Lab. Sci., 1995, 32: 265). The complement system consists of more than 35 soluble and cell-bound proteins, 12 of which are directly involved in the complement pathways.

Examples of suitable chemotherapeutic agents include, but are not limited to, taxanes (*e.g*., docetaxel, paclitaxel), maytansines, duocarmycins, CC-1065, auristatins, calicheamincins and other enediyne anti-tumor antibiotics. Other examples include the anti-folates (*e.g*., aminopterin, methotrexate, pemetrexed, raltitrexed), vinca alkaloids (*e.g*., vincristine, vinblastine, etoposide, vindesine, vitorelbine), and anthracyclines *(e.g.,* daunorubicin, doxorubicin, epirubicin, idarubicin, mitoxantrone, vakubicin).

Examples of suitable enzymes include, but are not limited to, nucleolytic enzymes.

Examples of suitable radioisotopes include any α-, β- or γ-emitter which, when localized at a tumor site, results in cell destruction (S.E. Order, "Analysis, Results, and Future Prospective of the Therapeutic Use of Radiolaheled Antibody in Cancer Therapy", Monoclonal Antibodies for Cancer Detection and Therapy, R.W. Baldwin et al (Eds.), Academic Press, 1985). Examples of such radioisotopes include, but are not limited to, iodine-131 (¹³¹I), iodine-125 (¹²⁵I), bismuth-212 (²¹²Bi), bismuth-213 (²¹³Bi), astatine-211 (²¹¹At), rhenium-186 ⁽¹⁸⁶Re), rhenium-186 (¹⁸⁸Re), phosphorus-32 (³²P), yttrium-90 (⁹⁰Y), samarium-153 (¹⁵³Sm), and lutetium-177 (¹¹⁷Lu).

### II. Diagnostic Chlorotoxin Agents

As already mentioned above, a "diagnostic chlorotoxin agent" used in methods of diagnosis of the present invention comprises at least one chlorotoxin moiety associated with at least one labeling moiety. Labeling usually involves attachment or incorporation of one or more labeling moieties to the chlorotoxin moiety, preferably to non-interfering positions on the peptide sequence of the chlorotoxin moiety. Such non-interfering positions are positions that do not participate in the specific binding of the chlorotoxin moiety to tumor cells. In preferred embodiments, labeling does not substantially interfere with the desired biological or pharmacological activity of the chlorotoxin moiety.

The role of a labeling moiety is to facilitate detection of the diagnostic agent after binding to the tissue to be tested. Preferably, the labeling moiety is selected such that it generates a signal that can be measured and whose intensity is related to (*e.g*., proportional to) the amount of diagnostic agent bound to the tissue.

As described above in the case of chlorotoxin conjugates, the association between the chlorotoxin moiety and labeling moiety may be covalent or non-covalent. In case of covalent binding, the chlorotoxin and labeling moieties may be attached to each other either directly or indirectly. Linkages and methods of linkage described in the case of cytotoxic chlorotoxin conjugates can be used for diagnostic chlorotoxin agents.

In certain embodiments, the interaction between the chlorotoxin moiety and labeling moiety is non-covalent Examples of non-covalent interactions include, but are not limited to, hydrophobic interactions, electrostatic interactions, dipole interactions, van der Waals interaction, and hydrogen bonding. For example, a labeling moiety can be non-covalently attached to the chlorotoxin moiety by chelation (*e.g*., a metal isotope can be chelated to a polyHis region attached, *e.g.,* fused, to a chlorotoxin moiety.)

In certain embodiments, the chlorotoxin moiety is isotopically labeled (*i.e*., contains one or more atoms that have been replaced by an atom having an atomic mass or mass number different from the atomic mass or mass number usually found in nature). Alternatively or additionally, an isotope may be attached to the chlorotoxin moiety.

As can readily be appreciated by one skilled in the art, a diagnostic chlorotoxin agent used in methods of the present invention can comprise any number of chlorotoxin moieties and any number of labeling moieties, associated to one another by any number of different ways. The design of a diagnostic agent will be influenced by its intended purpose(s) *(e.g., in vitro, in* vivo, or *ex* vivo diagnostic), the properties that are desirable in the context of its use, and the method selected for the detection.

### A. Chlorotoxin Moieties

Diagnostic agents used in methods of diagnosis of the present invention comprise at least one chlorotoxin moiety, *i.e.,* a chlorotoxin, a biologically active subunit thereof or a biologically active derivative thereof. Chlorotoxin moieties described above for use in cytotoxic chlorotoxin conjugates are also suitable for use in diagnostic chlorotoxin agents.

### B. Labeling Moieties

A labeling moiety may be any entity that allows detection of the diagnostic chlorotoxin agent after binding to the tissue or system of interest. Any of a wide variety-of detectable agents can be used as labeling moieties in diagnostic chlorotoxin agents, A labeling moiety may be directly detectable or indirectly detectable. Examples of labeling moieties include, but are not limited to: various ligands, radionuclides (*e.g*., ³H, ¹⁴C , ¹⁸F, ¹⁹F, ³²P, ³⁵S, ¹³⁵I. ¹²⁵I, ¹²³I, ⁶⁴Cu, ¹⁸⁷Re, ¹¹¹In, ⁹⁰Y, ^{99m}Tc, ¹⁷⁷Lu), fluorescent dyes (for specific exemplary fluorescent dyes, see below), chemiluminescent agents (such as, for example, acridinum esters, stabilized dioxetanes, and the like), bioluminescent agents, spectrally resolvable inorganic fluorescent semiconductors nanocrystals *(i.e.,* quantum dots), metal nanoparticles (*e.g.*, gold, silver, copper and platinum) or nanoclusters, paramagnetic metal ions, enzymes (for specific examples of enzymes, see below); colorimetric labels (such as, for example, dyes, colloidal gold, and the like), and biotin, dioxigenin, haptens, and proteins for which antisera or monoclonal antibodies are available.

In certain embodiments, the labeling moiety comprises a fluorescent label. Numerous known fluorescent labeling moieties of a wide variety of chemical structures and physical characteristics are suitable for use in the practice of methods of diagnosis of the present invention. Suitable fluorescent dyes include, but are not limited to, fluorescein and fluorescein dyes (*e.g.*, fluorescein isothiocyanine or FITC, naphthofluorescein, 4',5'-dichloro-2',7-dimethoxy-fluorescin, 6-carboxyfluorescein or FAM), carbocyanine, merocyanine, styryl dyes, oxonol dyes, phycoerythrin, erythrosin, eosin, rhodamine dyes (*e.g*., carboxytetramethyl-rhodamine or TAMRA. carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (*e.g.*, methoxycoumarin, dialkylaminocoumarin, hydroxycoumarin and aminomethylocoumarin or AMCA), Oregon Green Dyes *(e,g.,* Oregon Green 488, Oregon Green 500, Oregon Green 514), Texas Red, Texas Red-X, Spectrum Red^{™}, Spectrum Green^{™}, cyanine dyes *(e.g.,* Cy-3^{™}, Cy-5^{™}, Cy-3.5^{™}, Cy-5.5^{™}), Alexa Fluor dyes (*e.g*., Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes *(e.g.,* BODIPY FL, BODIPY R6G, BODIPY TMR, BODIPY TR, BODIPY 530/550, BODIPY 558/568, BODIPY 564/570, BODIPY 576/589, BODIPY 5S1/591, BODIPY 630/650, BODIPY 650/665), IRDyes (*e.g*., IRD40, IRD 700, IRD 800), and the like. For more examples of suitable fluorescent dyes and methods for coupling fluorescent dyes to other chemical entities such as proteins and peptides, see, for example, *"The Handbook of filuorescent Probes and Research Praducets",* 9^{th} Ed, Molecular Probes, Inc., Eugene, OR. Favorable properties of fluorescent labeling agents include high molar absorption coefficient, high fluorescence quantum yield, and photostability. In certain embodiments, labelling fluorophores desirably exhibit absorption and emission wavelengths in the visible (*i.e*., between 400 and 750 mn) rather than in the ultravioiet range of the Spectrum (*i.e*., lower than 400 nm).

In certain embodiments, the labeling moiety composes an enzyme. Examples of suitable enzymes include, but are not limited, those used in an ELISA, *e.g.,* horseradish peroxidase, beta-galactosidase, luciferase, and alkaline phosphatase. Other examples include beta-glucuronidase, beta-D-glucosidase, urease, glucose oxidase plus peroxide and alkaline phosphatase. An enzyme may be conjugated to a chlorotoxin moiety using a linker group such as a carbodiimide, a diisocyanate, glutaraldehyde; and the like.

In certain embodiments, the labeling moiety comprises a radioisotope that is detectable by Single Photon Remission Computed Tomography (SPECT) or Position Emission Tomography (PET). Examples of such radionuclides include, but are not limited to, iodine-131 (¹³¹I), iodine-125 (¹²⁵I), bismuth-212 (²¹²Bi), bismuth-213 (²¹³Bi), astatine-221 (²¹¹At), copper-67 (⁶⁷Cu), copper-64 (⁶⁴Cu), rhenium-186 (¹⁸⁶Re), rhenium-186 (¹⁸⁸Re), phosphorus-32 (³²P), samarium-153 (¹⁵³,Sm), lutetium-177 (¹¹⁷Lu), technetium-99m (⁹⁹Tc), gallium-67 (⁶⁷Ga), indium-111 (¹¹¹ In), and thallium-201 (²⁰¹TI).

In certain embodiments, the labeling moiety comprises a radioisotope that is detectable by Gamma camera. Examples of such radioisotopes include, but are not limited to, iodioe-131 (¹³¹I), and technetium-99m (^{99m}Tc).

In certain embodiments, the labeling moiety comprises a paramagnetic metal ion that is a good contrast enhancer in Magnetic Resonance Imaging (MRI). Examples of such paramagnetic metal ions include, but are not limited to, gadolinium III (Gd³⁺), chromium II (Cr³⁺), dysprosium III (Dy³⁺), iron III (Fe³⁺), manganese II (Mn²⁺), and ytterbium III (Yb³⁺). In certain preferred embodiments, the labeling Moieties comprises gadolinium III (Gd³⁺). Gadolinium is an FDA-approved contrast agent for MRI, which accumulates in abnormal tissues causing these abnormal areas to become very bright (enhances) on the magnetic resonance image. Gadolinium is known to provide great contrast between normal and abnormal tissues in different areas of the body, in particular in the brain.

In certain embodiments, the labeling moiety comprises a stable paramagnetic isotope detectable by nuclear magnetic resonance spectroscopy (MRS). Examples of suitable stable paramagnetic isotopes include, but are not limited to, carbon-13 (¹³C) and fluorine-19 (¹⁹F).

### III Methods of Treatment

Methods of treatment of the invention include administration of an effective amount of a cytotoxic chlorotoxin conjugates or a pharmaceutical composition thereof to an individual with a tumor of neuroectodermal origin.

### A. Indications

Any tumor of neuroectodermal origin present in at human patient can generally be treated using a method of the present invention. In certain embodiments, the tumor of neuroectodermal origin affecting the patient is a member of the group consisting of gliomas, meningiomas, ependymomas, medulloblastomas, neuroblastomas, gangliomas, pheochromocytomas, melanomas, peripheral primitive neuroectodermal tumors, small cell carcinoma of the lung, Ewing's sarcoma, and metastatic tumors of neuroectodermal origin in the brain, As shown in the Examples section, TM-601 (either biotinylated or iodinated) was found to specifically associate with tissues from these tumors.

In certain embodiments, the tumor of neuroectodermal origin affects the brain of the patient. In certain preferred embodiments, the brain tumor is a glioma. About half of all primacy brain tumors are gliomas. There are 4 main types of glioma: astrocytoma (which is the most common type of glioma in both adults and children), ependymoma, oligodendroglioma, and mixed glioma. Gliomas can be classified according to their location: infratentorial (*i.e*., located in the lower part of the brain, found mostly children patients) or supratentorial (*i.e*., located in the upper part of the brain, found mostly adults patents).

Gliomas are further categorizes according to their grade, which is determined by pathologic evaluation of the tumor. The World Health Organization (WHO) has developed a grading system, from Grade I gliomas, which tend to be the least aggressive to Grade IV gliomas, which tend to be the most aggressive and malignant Examples of low grade (*i.e*., Grade I or Grade II) gliomas include, but are not limited to, pilocytic astrocytoma (also called juvenile pilocytic astrocytoma), fibrillary astrocytoma, pleomorphic xantroastrocytomoa, and desembryoplastic neuroephelal tumor. High-grade gliomas encompass Grade III gliomas (*e.g*., anaplastic astrocytoma, AA) and Grade IV gliomas (glioblastoma multiforme, GBM). Anaplastic astrocytoma is most frequent among men and women in theirs 30s-50s, and accounts for 4% of all brain tumors. Glioblastoma multiforme the most invasive type of glial tumor, is most common in men and women in their 50s-70s and accounts for 23% of all primary brain tumors. The prognosis is the worst for Grade IV gliomas, with an average survival time of 12 months. In certain embodiments, methods of the present invention are used for the treatment of high-grade gliomas.

Despite aggressive treatment, gliomas usually recur, often with a higher grade and sometimes with a different morphology. While recurrence varies, Grade IV gliomas invariably recur. Thus, in certain embodiments, methods of the present invention are used for the treatment of recurrent gliomas, in particular, recurrent high-grade gliomas.

Tumors of neuroectodermal origin that can be treated using a method of the present invention include tumors that are refractory to treatment with chemotherapeutics. The term *"refractory",* when used herein in reference to a tumor, refers to a tumor (and/or metastases thereof) that shows no or only weak anti-proliferative response (*i.e*., nor or only weak inhibition of tumor growth) after treatment with at least one chemotherapeutic agent. Thus, a refractory tumor cannot be treated at all or only with unsatisfying results with at least one (preferably standard) chemotherapeutic agents. The present invention, where treatment of refractory tumors is mentioned is to be understood to encompass not only (1) tumors where one or more chemotherapeutics have already failed during treatment of a patient, but also (2) tumors that can be shown to be refractory by other means, *e,g*., biopsy and culture in the presence of chemotherapeutics.

Patients that can receive a treatment according to the present invention generally include any patient diagnosed with a tumor of neuroectodermal origin. As will be recognized by one skilled in the art, different methods of diagnosis may be performed depending on the location of the tumor. In certain embodiments, the presence of a tumor of neuroectodermal origin is detected using a diagnosis method provided herein.

The presence of a brain tumor is usually suggested by imaging studies such as CT (Computed Tomography) or CAT (Computed Axial Tomography) brain scans, or MRI (Magnetic Resonance Imaging) brain scans. Positron-emission tomography (PET) scanning is an additional imaging test that may be used in the detection of brain tumors. However, the specific tumor type can only be determined after biopsy, which is generally obtained at the time of surgery. A biopsy may not be performed if the tumor is located within a critical area of the brain that cannot be safely approached by surgery. In these circumstances, using a procedure called a stereotactic needle biopsy, the tumour can be safety sampler by inserting a needle through the skull into the brain itself, using CT or MRI to accurately position the biopsy needle. Results of biopsy help establish the diagnosis and indicate whether the tumor is amenable to other treatments.

### B. Dosages and Administrations

In a method of treatment of the present invention, a cytotoxic chlorotoxin conjugates, or a pharmaceutical compositions thereof, will generally be administered in such amounts and for such a time as is necessary or sufficient to achieve at least one desired result For example, a conjugate can be administered in such amounts and for such a time that it kills cancer cells, reduces tumor size, inhibits or delay tumor growth or metastasis, prolongs the survival time of patients, or otherwise yields clinical benefits.

A treatment according to the present invention may consist of a single dose or a plurality of doses over a period of time. Administration may be one or multiple times daily, weekly (or at some other multiple day interval) or on an intermittent schedule. The exact amount of a cytotoxic chlorotoxin conjugate or pharmaceutical composition thereof, to be administered will vary frown subject to subject and will depend on several factors (see below).

Cytotoxic chlorotoxin conjugates, or pharmaceutical compositions thereof, may be administered using any route of administration effective for achieving the desired therapeutic effects. Administration may be local or systemic. Methods of administration include, but are not limited to, dermal, intradermal, intramuscular, intraperitoneal, intravenous, subcutaneous, intranasal, pulmonary, epidural, ocular, intracavitary and oral routes. Administration may also be performed for example, by infusion or bolus injection, or by absorption through epithelial or mucocutaneous linings (*e.g.*, oral, mucosa, rectal and intestinal mucosa, etc),

In certain embodiments, it may be desirable to administer a cytotoxic chlorotoxin conjugate locally to an area in need of treatment. For example, systemic administration of chemotherapeutic agents to treat brain tumors is often limited by the blood brain barrier impenetrability, As a result, effective therapeutic concentrations are difficult to achieve in the brain without causing systemic adverse effects. Thus, in certain embodiments where the tumor to be treated is located in the brain, the cytotoxic chlorotoxin conjugate is administered locally.

Local administration to the brain (or to a specific region of the brain) may be achieved, for example, and not by way of limitation, by local infusion (*e.g*., during surgery), by infection (e,g., intracerebroventricular injection), by means of a catheter, or by means of a ventricular reservoir or pump placed in the tumor cavity during surgery or implanted subcutaneously in the scalp and connected to the brain via an outlet catheter. Alternatively or additionally, local administration can be achieved via the use of an implant device (*e.g*., a wafer implant containing the active ingredient) or a drug depot that can be placed locally during surgery. Such systems provide sustained drug release.

Depending on the route of administration, effective doses may be calculated according to the body weight, body surface area, or organ size of the subject to be treated. Optimization of the appropriate dosages can readily be made by one skilled in the art in light of pharmacokinetic data observed in human clinical trials. The final dosage regimen will be determined by the attending physician, considering various factors which modify the action of the drugs, *e.g.,* the drugs specific activity, the severity of the damage and the responsiveness of the patients, the age, condition, body weight, sex and diet of the patient, the severity of any present infection, time of administration, the use (or not) of other therapies, and other clinical factors. As studies are conducted using cytotoxic chlorotoxin conjugates, further information will emerge regarding the appropriate dosage levels and duration of treatment.

Typical dosages comprise 1.0 pg/kg body weight to 100 mg/kg body weight For example, for systemic administration, dosages may be 100.0 ng/kg body weight to 10.0 mg/kg body weight. For direct (*i.e*., local) administration to the site via microinfusion, dosages may be 1 ng/kg body weight to 1 mg/kg body weight.

, More specifically, in certain embodiments where the cytotoxic chlorotoxin conjugate is administered locally, in particular in cases of intracavitary administration to the brain, dosing of the conjugate may comprise administration of one or more doses comprising about 0.01 mg to about 100 mg of chlorotoxin, *e.g*., from about.0-05 to about 50 mg, from about 0.1 mg to about 25 mg, from about 0.1 mg to about 10 mg or from about 0.1 mg to about 5 mg. For example, in certain embodiments, one or more doses of cytotoxic chlorotoxin conjugate may be administered that each contains about 1 mg, about 1.5 mg, about 2 mg, about 2.5 mg, about 3 mg, about 3.5 mg, about 4 mg, about 4.5 mg or about 5 mg of chlorotoxin. For example, in other embodiments, one or more doses of cytotoxic chlorotoxin conjugate may be administered that each contains about 0.1 mg, about 0.15 mg, about 0.2 mg, about 0,25 mg, about 0.3 mg, about 0.5 mg, about 0.4 mg, about 0.45 mg, about 0.5 mg, about 0.55 mg, about 0.6 mg, about 0.65 mg, about 0.7 mg, about 0.75 mg, about 0.8 mg, about 0.85 mg, about 0.9 mg, about 0.95 mg or about 1 mg of chlorotoxin. In such embodiments, a treatment may comprise administration of a single dose of cytotoxic chlorotoxin conjugate or administration of 2 doses, 3 doses, 4 doses, 5 doses, 6 doses or more than 6 doses. Two consecutive doses may be administered at 1 day interval, 2 days interval, 3 days interval, 4 days interval, 5 days interval, 6 days interval, 7 days interval, or more than 7 days interval (*e.g*., 10 days, 2 weeks, or more than 2 weeks).

In embodiments where the cytotoxic chlorotoxin conjugate comprises a ¹³¹-I radiolabeled chlorotoxin and administration is performed locally, dosing of the conjugate may comprise administration of one or more doses comprising about 2 mCi to about 200 mCi ¹³¹-I, *e.g* , about 5 mCi to about 100 mCi ¹³¹-I, or about 10 mCi to about 50 mCi ¹³¹-I. For example, one or more doses of ¹³¹-I radiolabeled chlorotoxin conjugate may be administered that each contains about 10 mCi ¹³¹-I, about 20 mCi ¹³¹-I, about 30 mCi ¹³¹-I, about 40 mCi ¹³¹-I or about 50 mCi ¹³¹-I. In such embodiments, a treatment may comprise administration or a single dose of ¹³¹-I radiolabeled chlorotoxin conjugate or administration of multiple doses, *e.g*., 2 doses, 3 doses, 4 doses, 5 doses, 6 doses or more than 6 doses. Two consecutive doses may be administered at 1 day interval, 2 days interval, 3 days interval, 4 days interval, 5 days interval, 6 days interval, 7 days interval or more than 7 days interval.

In embodiments where a ¹³¹-I radiolabeled chlorotoxin is used, the patient is preferably administered supersaturated potassium iodide prior to administration of the ¹³¹-I radiolabeled chlorotoxin (*e.g.,* 1 day, 2 days or 3 days before treatment according to the present invention). Administration of supersaturated potassium iodide blocks uptake of ¹³¹-I by the thyroid gland, thus preventing damage such as hypothyroidism.

### C. Combination Therapies

It will be appreciated that methods of treatment of the present invention can be employed in combination with additional therapies (*i.e.,* a treatment according to the present invention can be administered concurrently with, prior to, or subsequently to one or more desired therapeutics or medical procedures). The particular combination of therapies (therapeutics or procedures) to employ in such a combination regimen will take into account compatibility of the desired therapeutics and/or procedures and the desired therapeutic effect to be achieved.

For example, methods of treatment of the present invention can be employed together with other procedures including surgery, radiotherapy (*e.g*., γ-radiation neuron beam radiotherapy, electron beam radiotherapy, proton therapy, brachytherapy, systemic radioactive isotopes), endocrine therapy, hyperthermia, and cryotherapy, depending on the tumor to be treated.

In many cases of brain tumor, a treatment of the present invention will very often be administered after surgery. In the Treatment of brain tumor, the main goal of surgery is to achieve a gross-total resection, *i.e.,* removal of all visible tumor. One of the difficulties in achieving such a goal is that these tumors are infiltrative, *i.e*., they tend to weave in and out among normal brain structures. Furthermore, there is a great variability in the amount of tumor that can be safely removed from the brain of a patient. Removal is generally not possible if all or part of the tumor is located in a region of the brain controlling critical functions,

In many cases of brain tumor, a treatment of the present invention will often be administered in combination with (*i.e*., concurrently with, prior to, or subsequently to), radiotherapy. In conventional treatments, radiotherapy generally follows surgery. Radiation is generally given as a series of daily treatments (called fractions) over several weeks. This "fractionated" approach to administering radiation is important to maximize the destruction of tumor cells and minimize side effects on normal adjacent brain. The area over which the radiation is administered (called the radiation field) is carefully calculated to avoid including as much as normal brain as is feasible.

Alternatively or additionally, Methods of treatment of the present invention can be administered in combination-with other therapeutic agents, such as agents that attenuate any adverse ejects (*e.g.,* antiemetics) and/or with other approved chemotherapeutic drugs. Examples of chemotherapeutics include, but are not limited to, alkylating drugs (mechlorethamine, chlorambucil. Cyclophosphamide, Melphalan, Tfosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), to name a few. For a more comprehensive discussion of updated cancer therapies see, http://www.cancer.gov/, a list of the FDA approved oncology drugs at http://www.fda.gov/cder/cancer/druglistframe.htm, and The Merck Manual, Seventeenth Ed. 1999, the entire contents of which are hereby incorporated by reference.

Methods of the present invention can also be employed together with one or more further combinations of cytotoxic agents as part of a treatment regimen, wherein the further combination of cytotoxic agents is selected from: CHOPP (cyclophosphamide, doxorubicin, vincristine, prednisone, and procarbazine); CHOP (cyclophosphamide, doxorubicin, vincristine, and prednisone); COP (cyclophosphamide, vincristine, and prednisone); CAP-BOP (cyclophosphamide, doxorubicin, procarbazine, bleomycin, vincristine, and prednisone); m-BACOD (methotrexate, bleomycin, doxorubicin, cyclophosphamide, vincristine, dexamethasone, and leucovorin); ProMACE-MOPP (prednisone, methotrexate, doxorubicin, cyclophosphamide, etoposide, leucovorin, mechloethamine, vincristine, prednisone, and procarbazine); ProMACE-CytaBOM (prednisone, methotrexate, doxorubicin, cyclophosphamide, etoposide, leucovorin, cytarabine, bleomycin, and vincristine); MACOP-B (methotrexate, doxorubicin, cyclophosphamide, vincristine, prednisone, bleomycin, and leucovorin); MOPP (mechloethamine, vincristine, prednisone, and procarbazine); ABVD (adriamycin/doxorubicin, bleomycin, vinblastine, and dacarbazine); MOPP (mechloethamine, vincristine, prednisone and procarbazine) alternating with ABV (adriamycin/doxorabicin, bleomycin, and vinblastine); MOPP (mechloethamine, vincristine, prednisone, and procarbazine) alternating with ABVD (adriamycin/doxorubicin, bleomycin, vinblastine, and dacarbazine); ChlVPP (chlorambucil, vinblastine, procarbazine, and prednisone); IMVP-16 (ifosfamide, methotrexate, and etoposide); MIME (methyl-gag, ifosfamide, methotrexate, and etoposide); DHAP (dexamethasone, high-dose cytaribine, and cisplatin); ESHAP (etoposide, methylpredisolone, high-dose cytarabine, and cisplatin); CEPP(B) (cyclophosphamide, etoposide, procarbazine, prednisone, and bleomycin); CAMP (lomustine, mitoxantrone, cytarabine, and prednisone); CVP-1 (cyclophosphamide, vincristine, and prednisone), ESHOP (etoposide, methylpredisolone, high-dose cytarabine, vincristine and cisplatin); EPOCH (etoposide, vincristine, and doxorubicin for 96 hours with bolus doses of cyclophosphamide and oral prednisone), ICE (ifosfamide, cyclophosphamide, and etoposide), CEPP(B) (cyclophosphamide, etoposide, procarbazine, prednisone, and bleomycin), CHOP-B (cyclophosphamide, doxorubicin, vincristine, prednisone, and bleomycin), CEPP-B (cyclophosphamide, etoposide, procarbazine, and bleomycin), and P/DOCE (epirubicin or doxorubicin, vincristine, cyclophosphamide, and prednisone),

As will be appreciated by one skilled in the art, the selection of one or more therapeutic agents to be administered in combination with a method of treatment of the present invention will depend on the tumor of neuroectodermal origin to be treated.

For example, chemotherapeutic drugs prescribed for brain tumors include, but are not limited to, temozolomide (Temodar^{®}), procarbazine (Matulane^{®}), and lomustine (CCNU), which are taken orally; vincristine (Oncovin^{®} or Vincasar PFS^{®}), cisplatin (Platinol^{®}), carmustine (BCNU, BiCNU), and carboplatin (Paraplatin^{®}), which are administered intravenously; and mexotrexate (Rheumatrex^{®} or Trexall^{®}), which can be administered orally, intravenously or intrathecally (*i.e*., injected directly into spinal fluid). BCNU is also given under the form of a polymer wafer implant during surgery (Giadel^{®} wafers). One of the most commonly prescribed combination therapy for brain tumors is PCV (procarbazine, CCNU, and vincristine) which is usually given every six weeks.

In embodiments where the tumor of neuroectodermal origin is a brain tumor, a method of the present invention may be used in combination with agents for the management of symptoms such as seizures and cerebral edema. Examples of anticonvulsants successfully administered to control seizures associated with brain tumors include, but are not limited to, phenytoin (Dilantin^{®}), Carbamazepine (Tegretol^{®}) and divalproex sodium (Depakote^{®}). Swelling of the brain may be treated with steroids (*e.g*., dexamethason (Decadron^{®})).

### D. Pharmaceutical Compositions

As mentioned above, methods of treatment of the present invention include administration of a cytotoxic chlorotoxin conjugate per se or in the form of a pharmaceutical composition. A pharmaceutical composition will generally comprise an effective amount or at least one cytotoxic chlorotoxin conjugate and at least one pharmaceutically acceptable carrier or excipient.

Pharmaceutical compositions may be formulated using conventional methods well-known in the art. The optimal pharmaceutical formulation can be varied depending upon the route of administration and desired dosage. Such formulations may influence the physical state, stability, rate of *in vivo* release, and rate of *in vivo* clearance of the administered compounds. Formulation may produce solid, liquid or semi-liquid pharmaceutical compositions.

Pharmaceutical compositions may be formulated in dosage unit form for ease of administration and uniformity of dosage. The expression "unit dosage form", as used herein, refers to a physically discrete unit of conjugate for the patient to be treated. Each unit contains a predetermined quantity of active material calculated to produce the desired therapeutic effect. It will be understood, however, that the total dosage of the composition will be decided by the attending physician within the scope of sound medical judgement

As mentioned above, in certain embodiments where a brain tumor is to be treated, the cytotoxic chlorotoxin conjugate is performed by intracavitary administration through injection or infusion. Pharmaceutical compositions suitable for administration by injection or infusion may be formulated according to the known art using suitable dispersing or wetting agents, and suspending agents. The pharmaceutical composition may also be a sterile injectable solution, suspension or emulsion in a non-toxic diluent or solvent, for example, as a solution in 2,3-butanediol. Among the acceptable vehicles and solvents that may be employed are water, Ringer's solution, U.S.P. and isotonic sodium chloride solution. In addition, sterile, fixed oils are conventionally employed as a solution or suspension medium. For this purpose, any bland fixed oil can be used including synthetic mono- or di-glycerides. Fatty acids such as oleic acid may also be used in the preparation of injectable formulations.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Liquid pharmaceutical compositions which are sterile solutions or suspensions can be administered by local infusion during surgery, by intracavitary injection (*e.g*., using a catheter or through a ventricular reservoir or pump placed during surgery or implanted subcutaneously in the scalp). Such reservoirs or pump are capable of delivering drugs as a constant infusion over an extended period of time.

In order to prolong the effect of a drug, it is often desirable to slow the absorption of the drug from injection. This may be accomplished by dissolving or suspending the active ingredient in an oil vehicle injectable depot forms are made by forming micro-encapsulated matrices of the drug in biodegradable polymers such as polylactide-polyglycolide. Depending on the ratio of drug to polymer and the nature of the particular polymer employed, the rate of drug release can be controlled. Depot injectable formulations can also be prepared by entrapping the drug in liposomes or microemulsions which are compatible with body tissues.

As mentioned above, in certain embodiments where a brain tumor is to be treated, the cytotoxic chlorotoxin conjugate embedded in an implant system (*e.g*., a wafer implant) is placed locally during surgery, thereby providing sustained release of the drug over a period of time. Thus, in certain embodiments, pharmaceutical compositions of cytotoxic chlorotoxin conjugates are obtained by incorporating or entrapping the active molecule into particles or a film or wafer of a biocompatible polymeric material (such as for example polyesters, polyamino acids, polyvinyl pyrrolidone, hydrogels, polylactic acid, ethylene vinylacetate, methylcellulose, hydroxymethylcellulose, and carboxymethylcellulose). Similar to the procedures used in the case of Gliadel^{®} Wafer, several wafers may be inserted into the tumor cavity at the time of surgery and left there to dissolve over a period of time.

### IV. Methods of Diagnosis

In another aspect, the present invention provides methods for the diagnosis of tumors of neuroectodermal origin. More specifically, methods are provided for differentiating neuroectodermal tumor-derived neoplastic tumor tissue from non-neoplastic tissue. Such methods include contacting a tissue of interest with a diagnostic chlorotoxin agent described herein, and measuring the binding of the diagnostic agent to the tissue of interest relative to normal tissue. An elevated level of binding, relative to normal tissue, indicates that the tissue of interest is neoplastic.

### A. Tissue Binding

The tissue to be tested according to methods of diagnosis of the present invention may be a fresh or frozen tissue sample collected from a patient, or an archival tissue sample with known diagnosis, treatment and/or outcome history. The tissue may originate from a live patient (*e.g*., obtained by biopsy) or from a deceased patient (*e.g*., obtained at autopsy). Alternatively, methods of the present invention may be used to detect and localize the presence of a neoplastic tissue *in vivo* (*i.e*., within a living patient).

Contacting of the tissue of interest with a diagnostic chlorotoxin agent is preferably carried out under conditions (*e.g*., concentration of diagnostic agent? contacting time and conditions) that allow the diagnostic agent to interact with the tissue so that the interaction results in specific binding of the agent to the tissue, if the tissue is neoplastic and of neuroectodermal origin. The contacting may be carried out by any suitable method known in the art. For example, for *in vivo* or *ex vivo* tissue samples, the contacting may be carried out by incubation (see, for example, Examples 5-16).

For *in vivo* methods of diagnosis, an effective amount of a diagnostic chlorotoxin agent, or a pharmaceutical composition thereof, is administered to the patient. Administration may be performed by any suitable method that results in interaction between the diagnostic agent and tissue of interest such that specific binding of the diagnostic agent occurs if the tissue is a neuroectodermally-derived neoplastic tissue. Administration may be systemic or local. In embodiments where the tissue to be tested is located in the brain, intracavitary administration of the diagnostic agent may be carried out, as described above (see also Examples 17 and 18).

Generally, the dosage of a diagnostic chlorotoxin agent will vary depending on considerations such as age, sex, and weight of the patient, area(s) of the body to be examined, as well as the administration route. Factors such as contraindications, concomitant therapies, and other variables are also to be taken into account to adjust the dosage of the diagnostic agent to be administered. This can, however, be readily achieved by a trained physician. In general, a suitable dose of a diagnostic chlorotoxin agent corresponds to the lowest amount of diagnostic agent that is sufficient to allow detection of neuroectodermally-derived tumor tissue in the patient. In certain embodiments, to minimize this dose, it is preferred that administration be local, for example intracavitory administration in the case of brain tissue.

After contacting of the tissue of interest with the diagnostic chlorotoxin agent and after sufficient time has elapsed for the interaction to take place, binding of the diagnostic agent is measured.

### B. Binding Detection

As will be recognized by one skilled in the art, detection of the binding of the diagnostic chlorotoxin agent to the tissue of interest may be carried out by any of a wide variety of methods including, but not limited to, spectroscopic, photochemical, biochemical, immunochemical, electrical, optical or chemical means. Selection of a detection method will generally be based on the nature of the labeling moiety of the diagnostic agent *(i.e.,* fluorescent moiety, enzyme, radionuclide, paramagnetic metal ion, and the like) and on the nature of the tissue sample (*i.e*., *ex vivo* or *in vivo* tissue sample).

For example, in embodiments where the labeling moiety comprises an enzyme, binding may be detected using colorimetric, spectrophotometric, fluorospectrophotometric, amperometric or gasometric techniques, as is well-known in the art.

In embodiments where the labeling moiety comprises a fluorescent compound, binding may be detected using fluorescence detection techniques, including, but not limited to, conventional fluorescence microscopy, confocal fluorescence microscopy, two-photon fluorescence microscope, fluorescence multi-well plate readers, and fluorescence activated cell sorters (FACS). The fluorescence signal from a diagnostic chlorotoxin agent bound to a tissue can also be visualized after DBA (diaminobenzidine) photoconversion. Photoconversion is the process by which a fluorescent probe is converted into an electron-dense probe, labeling the area or structure of interest for study at light and electron microscope levels (A.R. Maranto, Science, 1982, 217: 953-955; H.C. Mutasa, Biotech, Histochem., 1995, 70: 194-201).

In embodiments where the labeling moiety is a radionuclide or paramagnetic metal ion, detection of the binding may be performed using an imaging technique. Such imaging techniques are particularly useful for the detection of binding in an *in vivo* tissue. Different imaging techniques can be used depending on the nature of the labeling moiety. For example, the binding may be detected using Magnetic Resonance Imaging (MRI) if the labeling moiety comprises a paramagnetic metal ion (*e.g*., Gd³⁺). Single Photon Emission Computed Tomography (SPECT) and/or Positron Emission Tomography (PET) can be used for binding detection if the labelling moiety comprises a radioisotope (*e.g*., ¹³¹I, and the like).

### C. Diagnosis

According to diagnostic methods of the present invention, a tissue is identified as a neuroectodermally-derived neoplastic tissue if the level of binding of the diagnostic chlorotoxin agent to the tissue of interest is elevated compared to the level of binding of the diagnostic chlorotoxin agent to a normal tissue. As already mentioned above, a normal tissue is herein defined as a non-neoplastic tissue.

In preferred embodiments, the levels of binding to the tissue of interest and to the normal tissue are determined using the same method (*i.e*., same diagnostic chlorotoxin agent under substantially the same contacting conditions, and same detection method). In certain preferred embodiments, the normal tissue is of the same nature as the tissue being tested (*e.g*., if the tissue of interest is from brain tissue, the normal tissue is also from brain tissue). The tissue to be tested and the normal tissue may be from the same patient. For example, when the method is performed *in vivo*, the level of binding of the diagnostic agent measured in a region of an organ of interest (*e.g*., the brain) may be compared to the level of binding of the diagnostic agent measured in a normal region of the same organ. Alternatively, the tissue to be tested and the normal tissue may originate from the patient to be tested and a healthy individual, respectively.

In certain embodiments, the tissue of interest is identified as a neuroectodermally-derived neoplastic tissue if the level of binding measured is higher than the level of binding to a normal tissue. For example, the level of binding may be at least about 2 times higher, at least about 3 times higher, at least about 4 times higher, at least about 5 times higher, at least about 10 times higher, at least about 25 times higher, at least about 50 times higher, at least about 75 times higher, at least about 100 times higher, at least about 150 times higher, at least about 200 times higher, or more than 200 times higher than the level of binding to a normal tissue.

### Examples

The following examples describe some of the preferred modes of making and practicing the present invention. However, it should be understood that these examples are for illustrative purposes only and are not meant to limit the scope of the invention. Furthermore, unless the description in an Example is presented in the past tense, the text, like the rest of the specification, is not intended to suggest that experiments were actually performed or data were actually obtained.

### EXAMPLE 1: Summary of Chlorotoxin Results from Glioma Experiments

Recent studies have demonstrated that a common receptor is expressed by the vast majority of glioma cells. This antigen is targeted by chlorotoxin (Ctx or TM-601), a 36 amino acid peptide originally isolated from Leiurus quinquestriatus scorpion venom. Chlorotoxin selectively binds to the membrane of glioma cells allowing selective targeting of these cells within the CNS (L. Soroceanu et al., Cancer Res., 1998, 58: 4871-4879). The antigen targeted by this peptide has not yet been unequivocally identified at the molecular level. Thus far, the data indicates that chlorotoxin binds to a membrane protein of 72 kDa molecular weight that is preferentially expressed in the cytoplasmic membrane of glioma cell. Binding of the peptide enhances glioma cell proliferation (N. Ullrich and H. Sontheimer, Am. J. Physio., 1997, 273: C1290-1297) and inhibits the ability of glioma cells to migrate in Transwell assays, an *in vitro* assay to evaluate tumor invasiveness (L. Soroceanu et al., J. Neuroscience, 199, 19: 5942-5954). Chlorotoxin appears to exert these effects by reducing the membrane permeability to CR ions thereby preventing cell volume changes that are required to allow cells to invade healthy tissue (L. Soroceanu et al., J. Neuroscience, 199, 19: 5942-5954; ; McFerrin and Sontheimer, Neuron. Glia Biology, 2005, 2: 39-49).

### EXAMPLE 2: Immunohistochemical Staining of Gliomas with Chlorotoxin

Over 250 frozen or paraffin sections of human biopsy tissues were histochemically stained with a chemically synthesized form of chlorotoxin containing a detectable biotin group chemically attached to the N terminus (TM-601). Binding of the TM-601 molecule was observed on selective cells associated with essentially-all glioma tumors with up to 95% positive cells per tumor. Based on these studies, it was proposed to utilize chlorotoxin as a glioma specific marker and as a potential therapeutic tool for targeting glioma tumors. For such purposes, chlorotoxin linked to radioactive molecules or cytotoxic moieties such as saporin could be employed.

### EXAMPLE 3: Recombinant DNA Manipulation of Chlorotoxin

Using techniques well known in the art, one may prepare recombinant proteins specifically engineered to mimic the binding and action of the native toxin. The biological activity of the synthetic chlorotoxin is as effective for chloride ion channel blockade as the native venom toxin. Recombinant techniques are used to synthesized chlorotoxin in *E coli* using a modified PGEX vector system and the toxin may be linked to various fusion proteins using common restriction sites. After synthesis of recombinant chlorotoxin, it may be linked to carious cytotoxic proteins including glutathione-S-transferase (GST), gelonin, ricin, diptheria toxin, compliment proteins and radioligands and other such proteins as are Well known in the immunotoxin art to form a fusion protein.

### EXAMPLE 4: Rationale for the Examination of Neuroectodermally Derived Tumors for Chlorotoxin Binding

Given the similarities that can be shared between gliomas and other neuroectodermally-derived cells, and the arguments developed that propose similar propensities of neuroectodermal cells to migrate, a thorough investigation was undertaken to examine neuroectodermally derived tumors for the expression of chlorotoxin binding sites.

### EXAMPLE 5: Preparation of Sections from Frozen or Paraffin-Embedded Human Biopsies

Most of the samples of human tissue, from both sexes, of different ages and races were obtained through the Cooperative Human Tissue Network, Tissue Procurement at UAB, UAB hospitals and the Human Brain Tissue Bank in London, Canada. Snap frozen tissue and fresh tissue embedded in a freezing gel were sliced at 8 microns and picked up onto positively charged glass slides. The sections were then fixed in 4% paraformaldehyde or milloniqs according to the staining protocol. Paraffin blocks were sectioned and prepared according to standard procedures.

### EXAMPLE 6: Examination of Biopsy Samples for Chlorotoxin Binding

Biopsy sections were blocked for 1 hour in 10% normal goat serum in PBS and treated with a dilution of biotinylated chlorotoxin overnight at 4°C. After thorough rinsings, the stainings were developed by avidin-biotin complex (ABC) technique (Vectastain Elite ABC Kit from Vector Laboratories, Burlington, Calif.) and visualized by the colorimetric reaction of DAB (3'3'-diaminobenzidine; Vector Laboratories) with the ABC complex.

The biopsy sections were counterstained with methyl green, a nuclear dye, to more easily visualize the unstained cells, Non-specific background label can vary from experiment to experiment due to changes in the effective concentrations of the label, condition of the tissue or the duration of the reaction. Therefore, a control section was Identically stained with methyl green but without the biotinylated chlorotoxin. Positive cell staining is identified by chlorotoxis-labeling above background when compared to its individual control of a successive slice.' Cells containing high amounts of endogenous peroxidase exhibit dark background staining in the controls due to the reaction of DAB with the peroxidases.

Finally, a third adjacent section was stained with both hematoxylin, a cell nuclei specific stain, and eosic, a cytoplasmic stain. Therefore, for each tissue analyzed, three adjacent sections were stained. These are shown in photomicrographs providing evidence of the specificity of Tom-601 chlorotoxin binding to tumors of neuroectodermal derivation in comparison to controls. In the photomicrographs, adjacent sections are identified as follows: Tom-601: biotinylated chlorotoxin detected by a brown reaction product of DAB with the biotin and further counterstained with methyl green; Control: the control section stained with only methyl green; and, H&E: the hematoxylin and eosin stained section.

### EXAMPLE 7: Glioblastoma Multiforme (GBM) Tumours

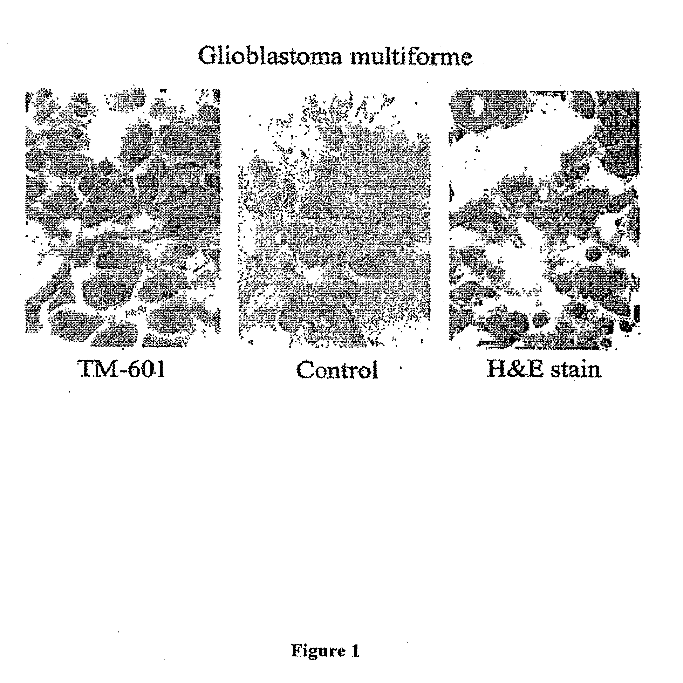

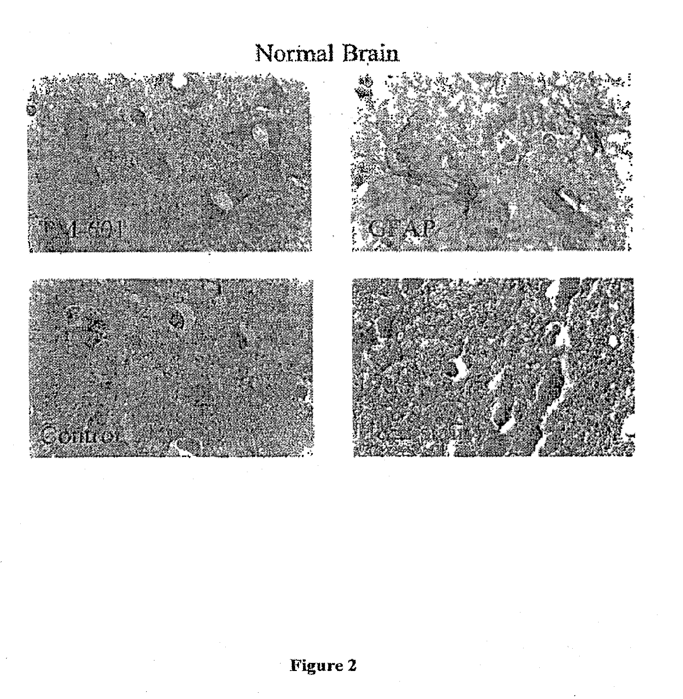

Glioblastoma multiforme (GBM) tumors were stained with the biotinylated chlorotoxin (TM-601). These tumors are extremely reactive to biotinylated chlorotoxin as all (31 out of 31) patient samples tested positive, as shown in FIG. 1. The result obtained with glioblastoma multiforme can be compared to the staining of the normal human brain tissue with biotinylated chlorotoxin (39/47 negative). FIG. 2. shows a representative staining. Normal brain tissue demonstrates a lack of TM-601 staining. This is consistent with earlier evidence of specificity of chlorotoxin binding to gliomas.

### EXAMPLE 8: GFAP Staining in the Normal Brain Tissue

The biopsy section was blocked for I hour in 10%.normal goat serum in PBS and then stained with antibodies against glial fibrillary acidic protein (GFAP; DAKO corporation, Carpinteria Calif.) overnight The secondary antibody conjugated to peroxidase was applied to the rinsed tissue for 2 hours, and the tissue was rinsed again before the stain was visualized with DAB. A typical glial fibrillary acidic protein stain of normal brain is shown in FIG. 2. Normal brain was positive for glial fibrillary acidic protein staining where it stained the astrocytes typically present in normal tissue.

### EXAMPLE 9: Neuroblastomas

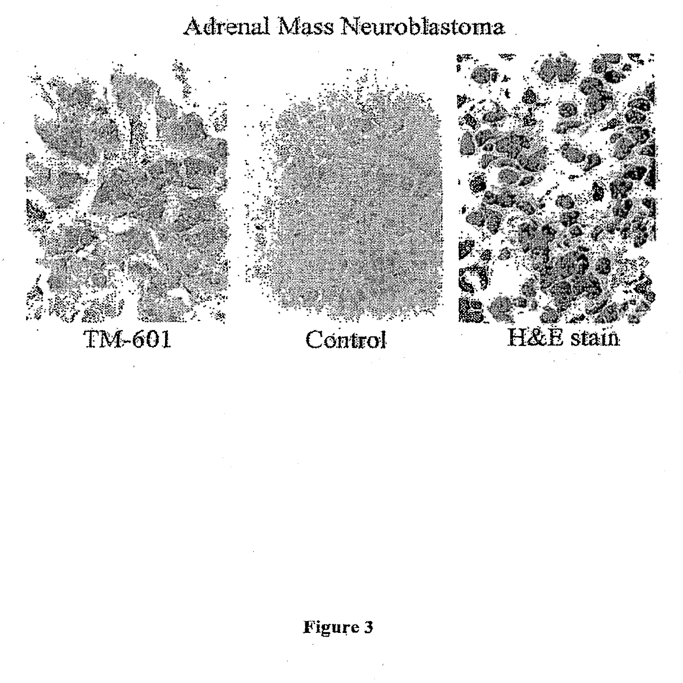

Neuroblastomas are tumors primarily found in children with a high incidence in the adrenals. Neuroblastomas show Tom-601 reactivity above the control staining, as seen in FIG. 3. Eight out of 9 neuroblastomas were positive for chlorotoxin binding.

### EXAMPLE 10: Pheochromocytomas

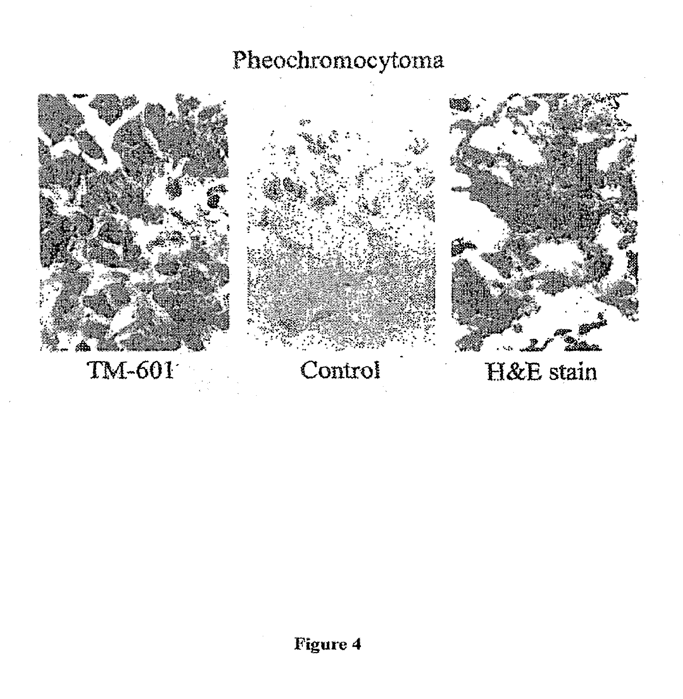

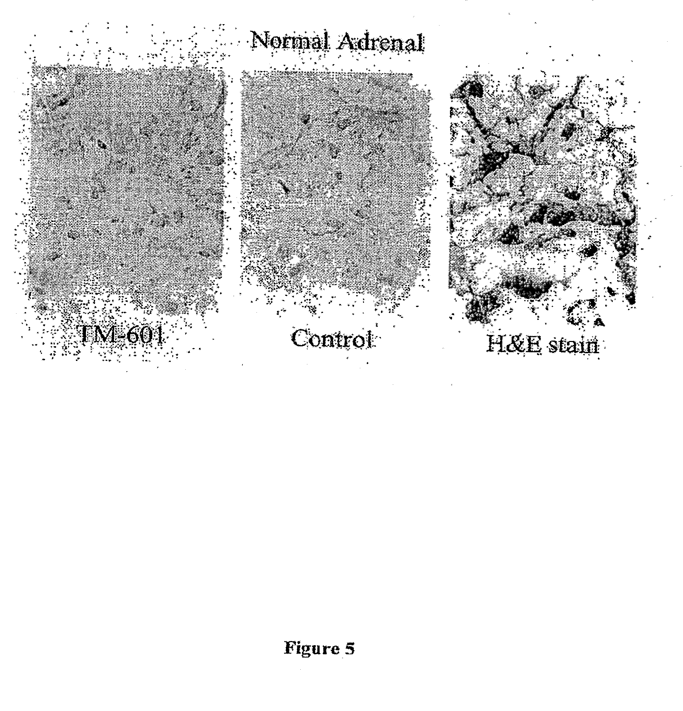

Pheochromocytomas are neoplastic chromaffin cells of the adrenal glands. This tumor was also found to show a high degree of staining, as seen in FIG. 4. Five out or six pheochromocytomas were positive for staining with biotinylated chlorotoxin, especially in comparison to TM-601 staining of the normal adrenals (3/3 negative) seen in FIG. 5.

### EXAMPLE 11: Melanomas

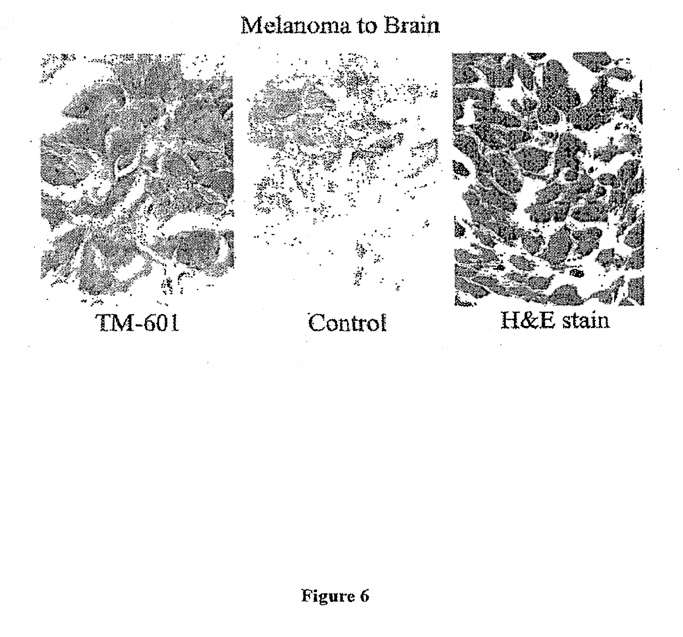

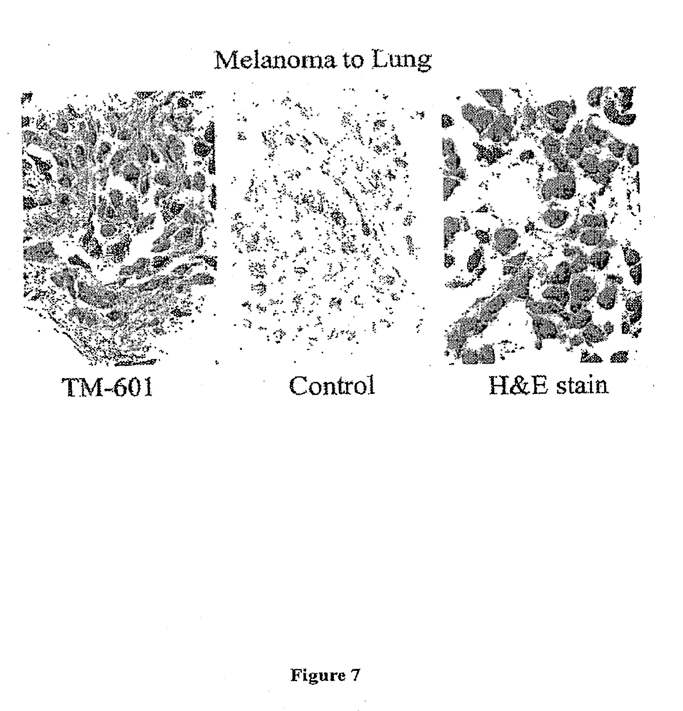

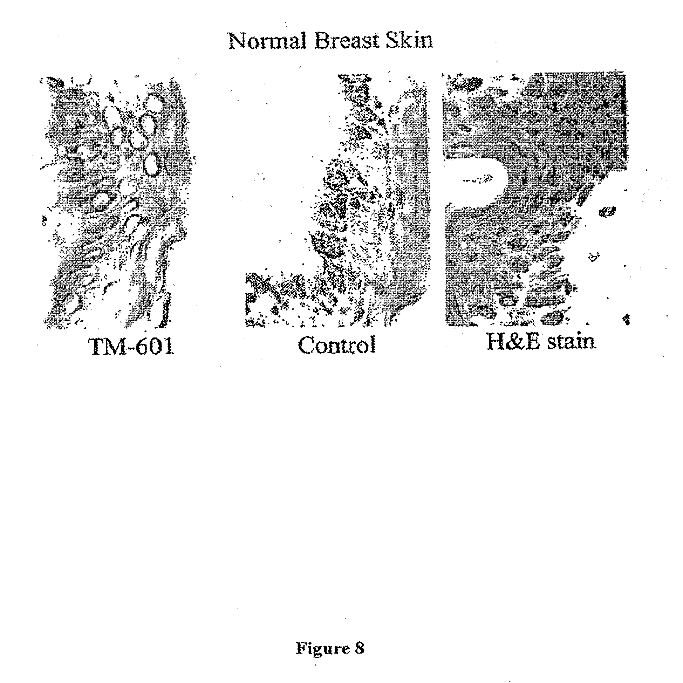

FIG. 6 shows the biotinylated chlorotoxin staining of a melanoma metastasized to the brain. Eleven out of 11 melanoma brain metastasis were positive for TM-601, and 5 out of 5 primary melanoma tumors were positive. In addition, melanoma metastasized to the lung were analyzed as seen in FIG. 7. Normal skin, however, is unreactive to TM-601 (6/6 negative) (FIG. 8) although there is some background staining in the melanocytes even in the controls.

### EXAMPLE 12: Small Cell Lung Carcinomas

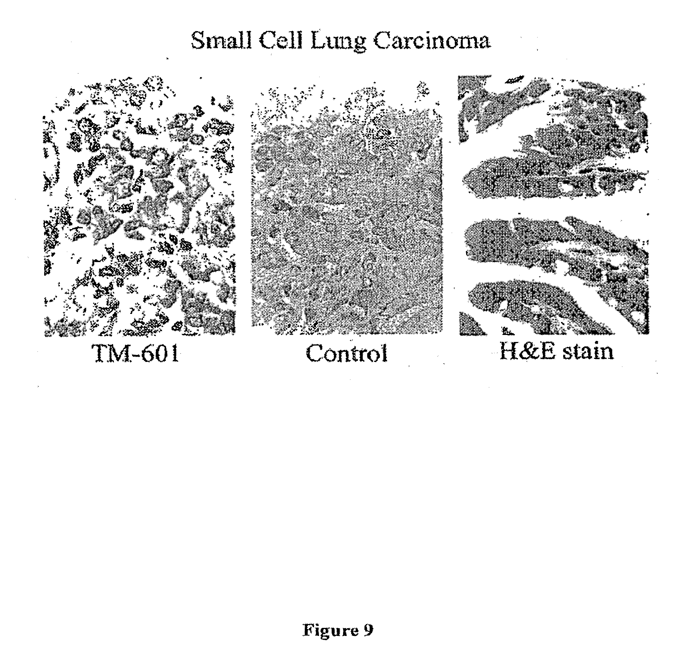

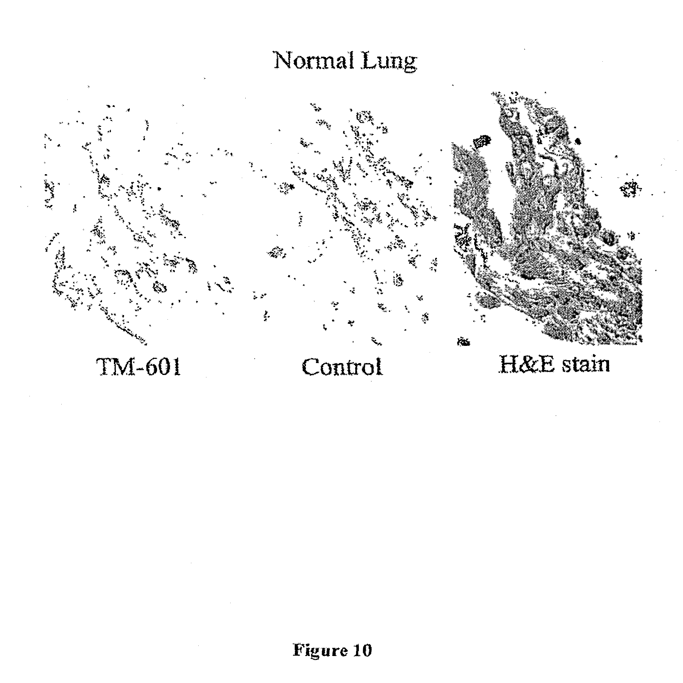

Small cell lung carcinomas are reactive to TM-601. There is good contrast between the cells that stain and those that do not (FIG. 9). The cells positive for TM-601 in the control (middle panel) are red blood cells which present high levels of background peroxidase stain. This TM-601 specificity can be further demonstrated by comparing the TM-601 staining of the small cell carcinoma (5/6 positive) and the normal lung (3/3 negative) (FIG. 10).

### EXAMPLE 13: Medulloblastomas

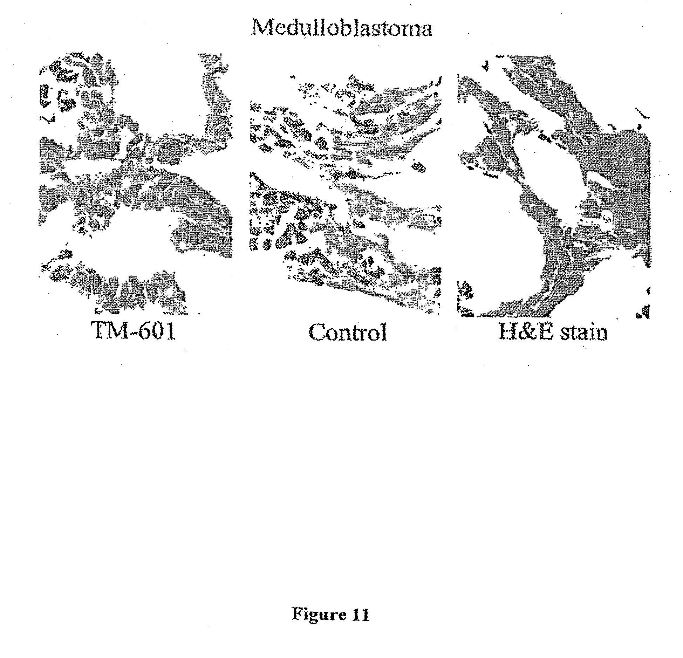

Another neuroectodermally derived tumor type are the medulloblastomas. They were found to exhibit specific reactivity to TM-601, as seen in FIG. 11 (4/4 positive).

### EXAMPLE 14: Ewing's Sarcoma

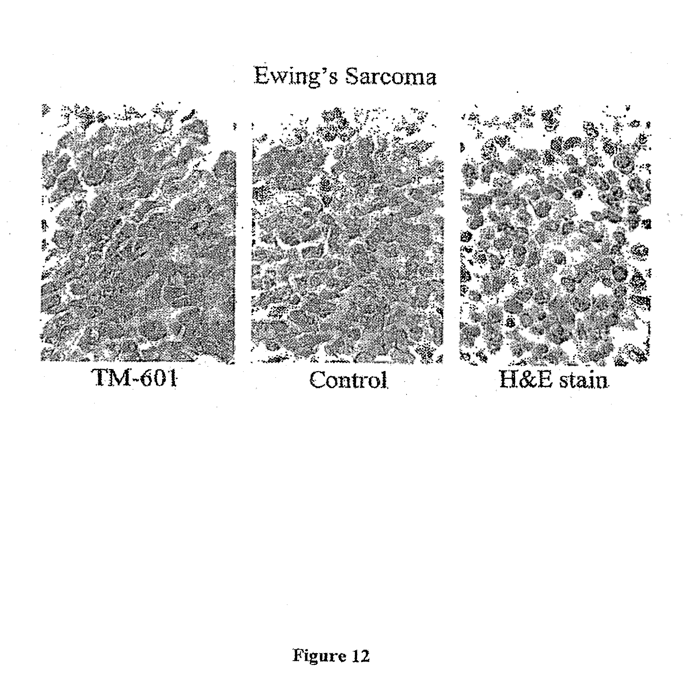

Ewing's sarcoma, a rare bone cancer sometimes found in soft tissue, was also found to be TM-601 positive (2/2) (FIG. 12).

### EXAMPLE 15: Testing of Potential Sites of Chlorotoxin Administration for Side Effects

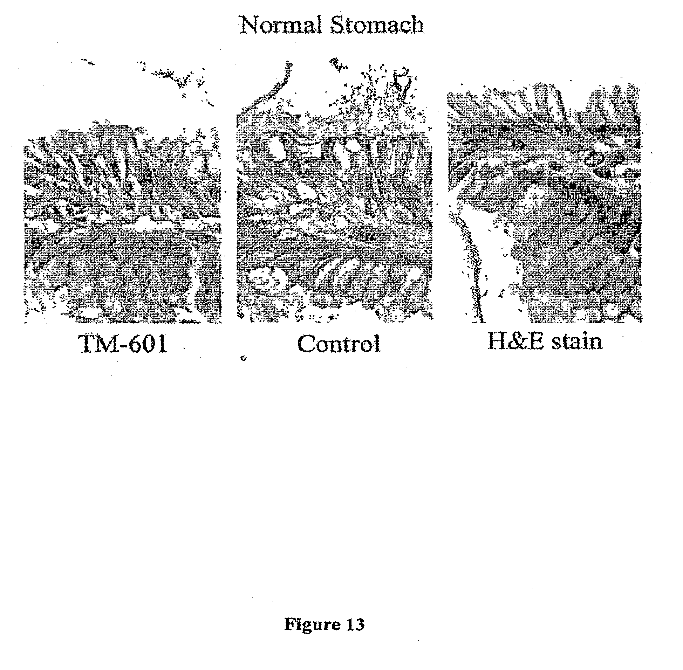

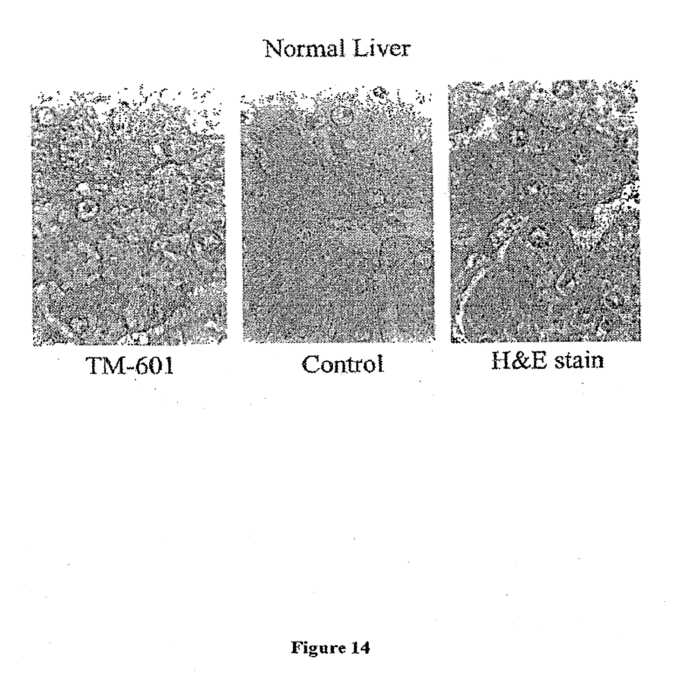

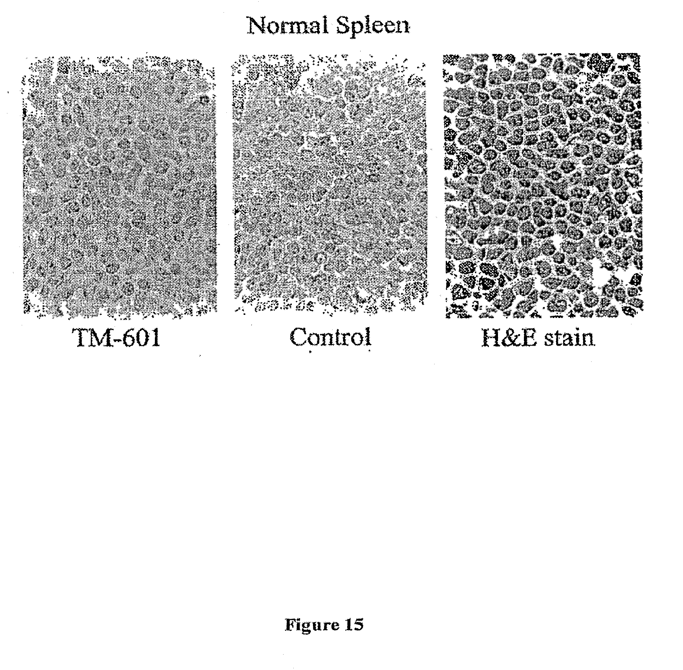

To aid in the design of drug therapy with this product, various normal tissues were stained with TM-601 to determine possible sites where side effects may occur. Preliminary evidence indicates that some of the most common targets for side effects such as the stomach and liver, are TM-601 negative (2/2 negative samples for both tissues, thus far) (FIG. 13 and FIG. 14 respectively). The staining of spleen tissue is also shown in FIG. 15 (2/2 negative). The TM-601 staining of other normal human tissues is summarized in Table 1.

### EXAMPLE 16: Summary of Tested Tumors and Tissues

As summarized in Table 1, the vast majority of neuroectodermally derived tumors were found to bind chlorotoxin, indicating that chlorotoxin has a more widespread utility to target tumors of neuroectodermal origin. Specifically, primitive neuroectodermnal tumors have been tested from 53 patients, 50 of which showed chlorotoxin specificity in the tumor material, as seen in Table 1. This staining was compared with the chlorotoxin staining of other types of CNS and PNS tumors as well as to various other normal human tissues.

TM-601 was found to specifically associate with neuroectodermally-derived tumors including medulloblastomas, neuroblastomas, ganglioneuromas, melanomas, pheochromocytomas, small cell lung carcinomas and Ewing's sarcomas. Thus, chlorotoxin-derived molecules can be utilized to specifically target the above-identified neuroectodermally-derived tumors, for therapeutic or diagnostic purposes. Likewise those tumors can also be targeted by other molecules such as antibodies that bind to the chlorotoxin receptor, presumed to be the 72 kD Cl⁻ ion channel.

### EXAMPLE 17: Treatment of patients with high grade glioma with ¹³¹I-TM-601

The Applicants have then evaluated the safety, tolerability, biodistribution and dosimetry of intracavitary administered ¹³¹I-TM-601 in patients with recurrent high grade glioma. Some results of this study were reported in A.N. Mamelak et al., J. Clin. Oncol., 2006,24:3644-3650; Hockaday et al., J. Nucl. Med., 2005,46: 380-586.

The goals of this study were primarily to evaluate the safety and toxicity of a single dose of intracavitary ¹³¹I-TM-601 infused into the tumor resection cavity and to evaluate biodistribution and dosimetry. Because TM-601 had never been given to humans and the toxic effects and dose-limiting toxicities of ¹³ Iodine are well documented, the Phase I trial focused on the potential toxic effects of escalating doses of peptide, not 131-Iodine. Pre-clinical data and theoretical calculations indicated that a 10mCi dose of 131-Iodine was potentially large enough to provide radiotherapy to patients, carried a small possibility of inducing a DLT, and was small enough to permit nuclear medicine imaging and biodistribution studies that were critical to the primary study endpoints. Estimates of the number of surface receptors for TM-601 on glioma cells indicated that peptide doses in the range of 0.1-1.0 mg were adequate to saturate all binding sites. Thus three peptide doses were chosen for dose escalation studies, with the amount of radioactivity fixed at 10 mCi to avoid confounding any effects of peptide, escalation with radioactivity escalation.

Preliminary assessment of anti-tumor effect was a secondary end point. Six patients were enrolled in one of three sequential dosing panels: Panel 1, with 0.25 mg of TM-601, Panel 2, with 0.50 mg of TM-601, or Panel 3, with 1.00 mg of TM-601, each radiolabeled with 10 mCi (± 10%) of ¹³¹L Treatment within a dosing panel would have been interrupted if two or more of the initial three patients experienced a Dose Limiting Toxicity (DLT) Grade III-or higher, (National Cancer Institute common toxicity criteria (NCI CTC) 3.0 graded as at least probably related to treatment). Dose escalation similarly would have been interrupted if two or more DLTs occurred within a single dosing panel. If two patients at a given dose experienced a DLT, the previous dose level would have been identified as the Maximum Tolerated Dose (MTD). Every patient was followed clinically for up to 180 days after the, dose of ¹³¹I-TM-601. All efficacy and safety analyses were performed on the intent-to-treat (ITT) cohort of all patients who received a single dose of intracavitary 131I-TM-601.

### Preparation of ¹³¹I-TM-601

TM-601 (lyophilized, sterile, and pyrogen-free) was radiolabeled with 10 mCi ¹³¹I via the Iodogen bead method (E. Ricotti et al., Blood, 1998, 91: 2397-2405) at the clinical site and used within 78 hours (typically < 2 hours). Release specifications required less than 5% free iodine (by instant thin layer chromatography) and no pyrogenicity, Every labeling remained sterile during the two week testing period.

### Patients and Treatment Protocol

Nineteen (19) patients with recurrent high-grade glioma were enrolled in the study; 18 with GBM and one with AA. One GBM patient was excluded after surgery due to persistently elevated transaminases that were present prior to surgery that lead to a diagnosis of previously undetected Hepatitis C. The demographics of the patient population are outlined in Table 2. All patients received at least one dose of study medication.

18 patients (17 GBM, 1 AA), 18 years of age or older with histologically documented recurrent supratentorial malignant gliomas, KPS ≥60%, eligible for cytoreductive craniotomy were enrolled in this trial. An intracavitary catheter with subcutaneous reservoir was placed in the resected tumor cavity during surgery. Two weeks post surgery patients received a single dose of 131I-TM-601 from one of three sequential dosing panels; 0.25 mg, 0.50 mg or 1.0 mg of TM-601, each labeled with 10 mCi of ¹³¹I.

**Table 2. Patient Characteristics**

| **Patient Characteristics** | | **Overall** | **Group 1** | **Group 2** | **Group 3** |
|---|---|---|---|---|---|
| **Number** | Female | 7 | 2 | 2 | 3 |
| | Male | 11 | 4 | 4 | 3 |
| **Age, mean, years (SD)** | | 47.2(10,6) | 44.5(14.7) | 46.3(9.6) | 50.7(10.7) |
| **Race** | Caucasian | 15 | 5 | 5 | 3 |
| | Hispanic | 1 | 0 | 0 | 1 |
| | Black | 1 | 0 | 0 | 2 |
| | Asian | 2 | 1 | 1 | 0 |
| **Karnofsky Performance (SD)** | | 82.8 (11.3) | 83.3 (15.1) | 81.7(11.7) | 83.3 (8.2) |
| **Histology** | GMB | 17 | 5 | 6 | 6 |
| | AA | 1 | 1 | 0 | 0 |
| **Tumor Location** | Temporal lobs, | 10 | 6 | 0 | 4 |
| | Parietal lobe | 50 | 0 | 4 | 1 |
| | Frontal lobe | 3 | 0 | 2 | 1 |
| **Prior Therapy** | Radiation | 18 | 6 | 6 | 6 |
| | hemotherapy | 18 | 6 | 6 | 6 |

Adult (>18 years) patients with histologically documented supratentorial malignant glioma, Kamofsky Performance Status (KPS; 11) ≥60%, life expectancy of at least 3 months, and adequate cardiac, renal, hepatic and hematologic function were eligible for this trial. Patients were required to have unifocal tumors that had progressed at the site of original disease following standard of care. Additional inclusion criteria included: tumor cross sectional diameter of less than six centimeters; no direct communication with the ventricle; no previous immunotherapy, implantable chemotherapy, or stereotactic radiosurgery; at least six weeks from the last dose of nitrosourea-containing chemotherapy. Patients with prior treatment with gene therapy, or with evidence of leptomeningeal disease, multicentric intraparenchymal or disseminating tumors by MRI were excluded from the study.

Eligible patients underwent surgery for tumor debulking; pathologic confirmation of recurrent high grade glioma was performed for all patients. During surgery, a ventricular access device (VAD) with subcutaneous reservoir (Rickham or Ommaya reservoir) was placed in the tumor cavity. Patients were allowed to recover from surgery for a period of 14-28 days prior to undergoing treatment with the study drug. This waiting period was chosen to avoid potential confounding between permanent neurological defects following surgery and adverse events attributed to the study drug. Patients underwent a neurological screening at 14-28 days after surgery to document any surgery-induced changes in function and were excluded from the trial if they suffered a neurological deficit or surgical complication that was deemed by the investigator to make proceeding with treatment unsafe, or if the KPS was <60%.

### Injection of Radiolabeled Peptide

Patients received supersaturated potassium iodide, 300 mg/kg, one day prior and three days after administration of ¹³¹I-TM-601 to block uptake of ¹³¹I by the thyroid gland. Prior to infusion of the ¹³¹I-TM-601, potency of the VAD was evaluated by injection of 0.5-1 mCi of ¹¹¹IN-DTPA followed by gamma camera imaging of the head. Serial gamma camera images were acquired every two minutes for 16 minutes to determine if the ¹¹¹In-DTPA was leaking from the cavity site. The amount of leakage was measured using total counts in a selected region of interest over the study period. If >30% of the injected material had leaked from the cavity site, administration of the study drug would have been aborted.

Patients then received 25% of the total dose *via* direct injection into the VAD, were observed for 5 minutes, and then received the remainder of the study dose. Patients were closely monitored during drug infusion, and re-evaluated on a daily basis during the immediate (Day 0-8) post-infusion period, seen again at Day 22, and then followed for up to 180 days. Biodistribution and elimination were determined by urine and blood measures of radioactivity. Twenty-four hour urine collections were performed over Days 1-2, 2-3, 3-4, and 4-6 or -8, with an aliquot from each twenty four hour period used to determine average amount of excreted radioactivity during that time period. Blood samples were collected 1,2, and 4 hours after the completion of the infusion, on Days 2, 3,4, and at the time of imaging on day 6 or 8.

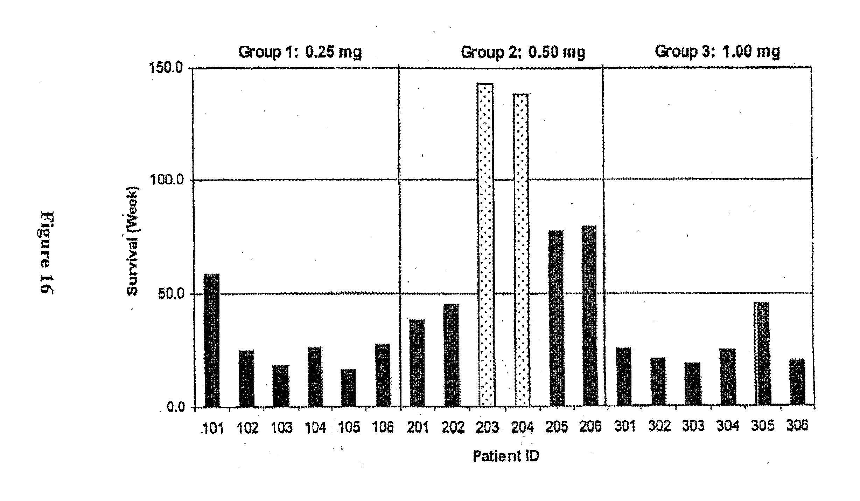

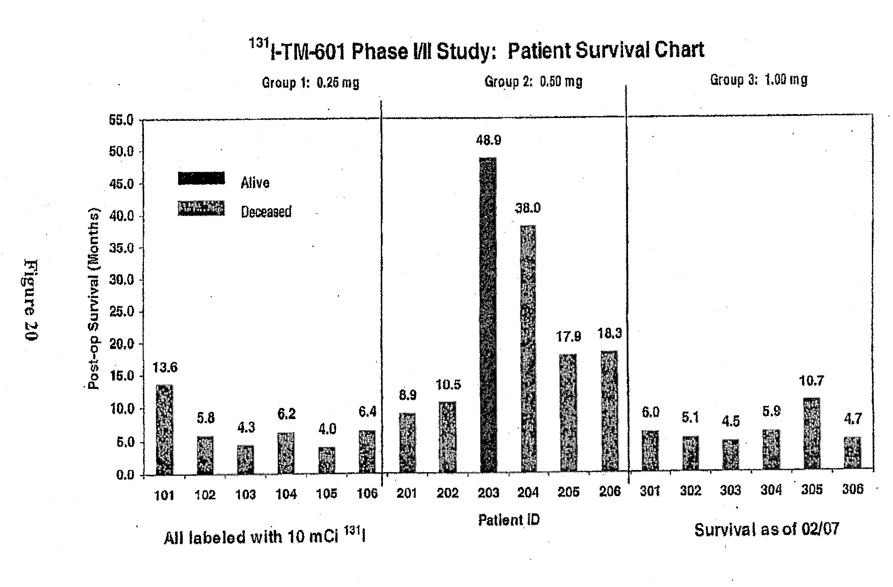

For unplanned reasons, two patients assigned to the 0.50 mg dose group and one patient assigned to the 1.00 mg group received a second dose of study medication. In one of these patients, SPECT images indicated that the first injection was accidentally delivered subcutaneously, and therefore did not enter the tumor resection cavity. Calculated radiation doses to normal organs following this subcutaneous infection were within the normal range of the inter-patient variation as demonstrated by the 17 other patients in the study and were determined to be clinically insignificant. This patient received a second injection of 10 mCi ¹³¹I-TM-601 into the reservoir, confirmed by subsequent SPECT images. Two other patients received a second dose on a "compassionate use" basis with approval of the FDA at 12 and 19 weeks following initial treatment FIG. 16 and FIG. 20 show survival from time of injection for all patients as of November 2005 and as of February 2007, respectively. Two patients demonstrated a small amount of ¹¹¹In-DTPA leakage into the ventricles and spinal fluid pathways. Radiation dose estimates suggest that the mediation dose of ¹³¹I-TM-601 to the spine would be in a range thought to be clinically insignificant (283 cGy and 378 eGy). In these cases the treating physician determined that administration of 10 mCi ¹³¹I-TM-601 was still appropriate for this study.

### Gamma Camera Imaging

*⁵⁷Co transmission scan.* A ⁵⁷Co transmission scan with and without the patient was used to obtain attenuation correction factors for total body image quantification, as previously described (D.C. Hockaday et al., J. Nucl- Med., 2005, 46: 580-556).

*Whole body and 2-dimensional brain SPECT scans:* After intracavitary injection of ¹³¹I-TM-601, anterior and posterior whole-body planar images and 2-dimensional (2D) SPECT scans were acquired as previously described (D.C. Hockaday et al., J. Nucl. Med., 2005, 46: 580-596). A 20 mL calibrated ¹³¹I source (∼100 µCi) was placed approximately 10 cm from the feet of the patient within the field of view. Subsequent images were acquired on days 1, 2, 3 and between 5 and 8 days post injection.

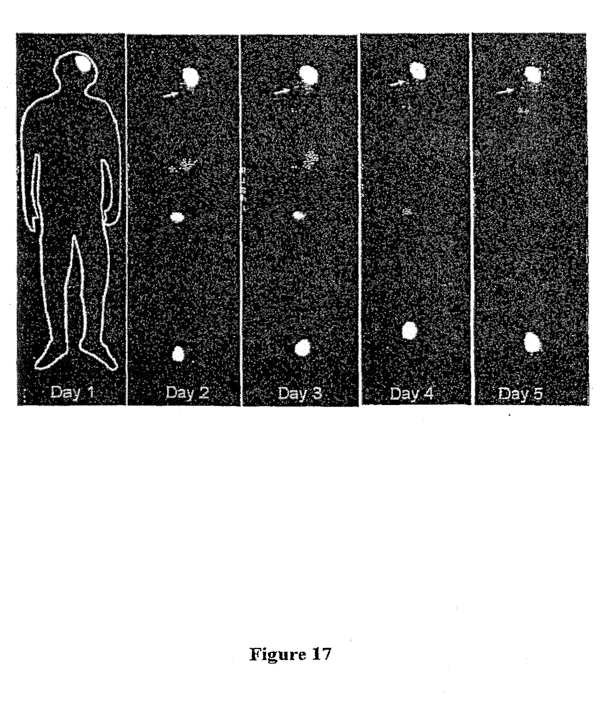

Gamma camera imaging showed the ¹³¹I-TM-601 localized to and remained primarily concentrated in and around the patient's surgical cavity for all five days imaged (a typical image is shown in FIG. 17.

### Magnetic Resonance Imaging(MRI)

Details of all imaging methods have been reported previously (D.C. Hockaday *et al.,* J. Nucl. Med., 2005,46: 580.586). Briefly, postoperative MRI scans were acquired for each patient prior to ¹³¹I-TM-601 injection. Sequences included 3 mm thick coronal, axial, and sagittal TI-weighted images (with and without gadolinium contrast), axial and coronal T2, axial FLAIR, and 3D-SPGR images. Additional images were acquired at 22 days following infection, and again at three and six months after treatment. The three week scan was performed to assess for any early inflammatory changes potentially related to the peptide, while later scans were done for surveillance of tumor status.

A detailed analysis of the imaging of this drug in the brain based on a subset of 9 patients has already been published (D.C. Hockaday et al., J. Nucl. Med., 2005, 46: 580-586), indicating that ¹³¹I-TM-601 diffuses into the brain at distances far greater than observed for antibodies and other large molecules (S.A. Grossman and J.F. Batara, Semin. Oncol., 2004, 31: 635-644). These observations suggest that TM-601 may be a useful means to deliver focused radiotherapy to patients with glioma. Limited by current state-of-the-art imaging technologies and because of the non-uniform microscopic distribution of ¹³¹I-TM-601 and residual tumor cells, the current macroscopic radiation dose calculations based on imaging may not accurately represent the actual radiation dose delivered to tumor cells (I.F. Parney and S.M. Chang, Cancer, 2003, 9: 149-156).

### Radiation Dosimetry

The tissue uptake, clearance and dosimetry of ¹³¹I-TM-601 for the whole body, normal organs and brain were determined based on five sequential, quantitative whole body gamma camera images (S. Shen et al., J. Nucl. Med., 2005, 46: 642-651). Organs with significant uptake of ¹³¹I-TM-601 were visualized above body background in whole-body images. The regions of interest (ROI) for these organs were contoured and counts in ROI were converted to radioactivity using imaging standards. Radiation to normal organs was calculated using the MIRDOSE III program based on the reference man (M.G. Sabin, J. Nncl. Med., 1996,37: 538-546). Localization of ¹³¹I- TM-601 to the tumor resection cavity and the radiation dose of ¹³¹I to, the tissue surrounding the resection cavity were evaluated based on these images. The counts in the SPECT images were converted to µCi of ¹³¹I based on a calibrated imaging standard. The distribution of ¹³¹I was converted to the radiation dose rate distribution using dose convolution with electron and photon dose-kernel. Radiation doses to the tumor resection cavity were estimated to within 2 cm of tumor margins because most recurrences occur within this distance of the original tumor volume (P.C. Burger et al., J. Neurosurg., 1988, 68:698-704; G.R. Harsh, Management of recurrent gliomas, in The Gliomas, M.S. Berger and C.B. Wilson (Eds), WB Saunders: Philadelphia, 1999, p. 649.).

Radioactivity concentrations in whole blood and urine were determined using a gamma well counter calibrated with a ¹³¹I standard. For blood, total radioactivity was calculated based on the area under the radioactivity-time curve, with the typical peak (µCi/mL). at 4 hours. Cumulated activity during the 0-4 hour window-was determined by trapezoid integration and cumulated activity > four hours was fitted with a mono-exponential curve. Marrow-to-blood ratio (G. Sgouros, J. Nucl. Med., 1993, 34: 689-694) was assumed to be 0.75, a value between 0.4 (typical for an intact antibody) and 1.0 (completely free) because of the small peptide size. Patient-specific marrow dose was estimated based on the electron radiation from the blood, the photon radiation from the remaining body and tumor cavity and the patient's body weight (S. Shen et al., J. Nucl. Med., 1999,40:2102-2106).

Results obtained are shown in Table 3 and Table 4. Radiation doses to normal organs were clinically insignificant (Table 3). In contrast, mean radiation dose to within 2 cm of the cavity wall was 81 cGy/mCi (median 49) and ranged from 12 to 275 cGy/mCi (Table 3). These values are substantially higher than doses to the whole body (mean 0.4 cGy/mCi) and to any other organs. Furthermore, the biological half-life of ¹³¹I-TM-601 in the tumor cavity margin was longer than in any other organ including the normal brain, indicating long-term retention of the drug in and around the injection site (Table 3). The median biological half-life in cavity margin was 70 (range 32-193) hours, 80 (range 25-86) hours, and 55 (range 41-62) hours for patients receiving 0.25, 0.50, and 1.0 mg peptide, respectively.

It was also of interest to look at the dose to the tumor cavity wall and the half-life of 131I-TM-601 in this area. These values are listed for each patient in Table 4.

**Table 3. Organ dose, half life and percent in organ at**

| **Organ** | **cGy/mCi(range)** | **T_{biol 1/2}, hours (range)** | **Percent in organ at Day 7** |
|---|---|---|---|
| Whole Body | 0.4(0.2-0.7) | 47(31-81) | 0.03 |
| Stomach | 0.7(0.2-1.3) | 25(17-40) | 0.019 |
| Kidney | 0.9 (0.3-1.8) | 26(18-39) | 0-013 |
| Thyroid | 5.3 (0.8-22.9) | NA* | NA* |
| Normal Brain | 1.1 (0.3-2.4) | 42(21-80) | 0.203 |
| Marrow (blood half- | 0.3(0.1-0.4) | 29 (9-41) | 0.013 |
| Bladder wall | 3,2 (1.5-6.5) | NA* | NA* |
| Tumor cavity wall | 81(12-275) | 63(25-193) | 3.37 |

| | | | |
|---|---|---|---|
| NA = not available | | | |

**Table 4. Dosimetry and half-life of ¹³¹I-TM-601 at tumor cavity wall.**

| **Dose(mg)** | **Patient ID** | **T_{1/2} bio(hr)** | **Average T_{1/2} bio,hr. (range)** | **Dose (cGy/mCi)** | **Average Dose, Gy/mCi(range)** |
|---|---|---|---|---|---|
| **0.25** | 101 | 32.2 | 67.5 (32.2-193.4) | 126 | 593 (126-2080) |
| | 102 | 63.5 | | 130 | |
| | 103 | 193.4 | | 805 | |
| | 104 | 40.2 | | 380 | |
| | 105 | 71.5 | | 2080 | |
| | 106 | 100.0 | | 2830 | |
| **0.50** | 201 | 86.4 | 79.8 (25.1-86.4) | 928 | 729(280-1296) |
| | 202 | 78.9 | | 350 | |
| | 203 | 55.2 | | 1296 | |
| | 204 | 25.1 | | 797 | |
| | 205 | 80.8 | | 662 | |
| | 206 | 85.5 | | 280 | |
| **1.00** | 301 | 52.3 | 55.4 (40.5-61.7) | 416 | 428 (345-1949) |
| | 302 | 57.5 | | 1948 | |
| | 303 | 46.8 | | 415 | |
| | 304 | 40.5 | | 345 | |
| | 305 | 60.3 | | 440 | |
| | 306 | 61.7 | | 550 | |

These data indicate a slightly longer half-life and higher radiation dose for patients receiving 0.50 mg of peptide compared with other groups, although this difference did not reach statistical significance.

### Histochemical Staining

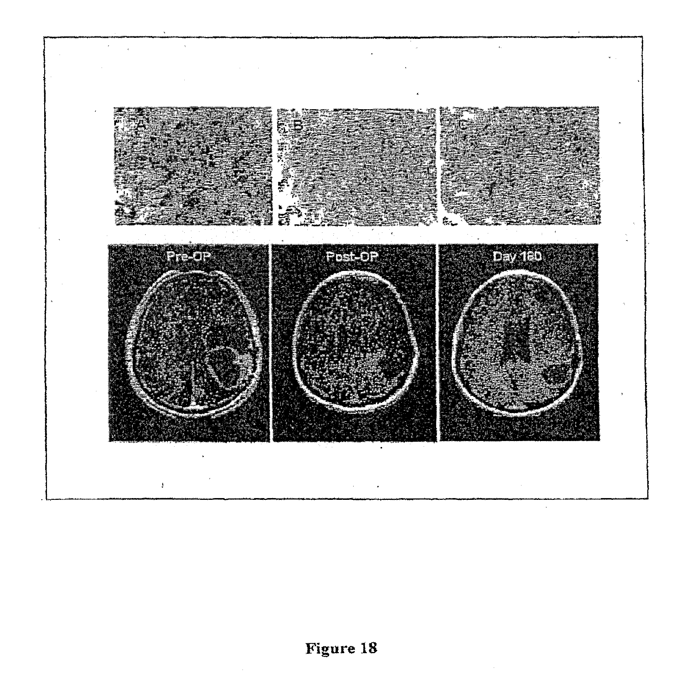

A tissue sample was obtained from each patient during surgery. Each specimen was subjected to immunohistochemical staining to test for TM-601 binding. Staining followed the method of Lyons et al. (Glia, 2002, 39: 162-173) with few modifications. Histochemistry of the tumor tissue from all patients stained intensely positive for TM-601 as represented in Figure 18A-C.

Biodistribution data of ¹³¹I.TM-601 indicated that this radiopeptide rapidly penetrated through the cavity wall with on average 79% or the radioactivity leaving the region of cavity within 24 hours after administration. The majority of the remaining radioactivity stayed tightly localized to the tumor cavity and surrounding regions, suggesting discrete binding to the tumor. Since animal models have demonstrated that injection of unbound radioactive iodine is essentially completely eliminated from a brain tumor site within 24 hours (unpublished data), this binding appears to be peptide and tumor specific. The amount of uptake and radiation doses in the stomach, kidneys, spleen and bladder were much lower compared to those reported in the literature for other modalities (Adult Brain Tumors (PDQ®): Treatment. Available at: http://www.nci.nih.gov/cancertopics/pdq/treatment/adultbrain/healthprofessional; accessed on March 16, 2005). There was no observable uptake of ¹³¹I-TM-601 in the small or large intestine at any time in any patient, suggesting that the excretion route of ¹³¹I-TM-601 is mainly through the urinary tract. Uptake of ¹³¹I-TM-601 in the thyroid was larger than in other solid organs, at 5.3 cGy/mCi (Range 0.8-22.9), but still far below levels required to damage the thyroid (doses in the range of 15 mCi have been reported to cause hypothyroidism in 15% of patients, and 100 Gy to cause hypothyroidism in greater than 50% of patients (Accelerate Brain Cancer Cure (ABC2) website. http://www.abc2.org/statistics.shtml, Accessed November 1, 2005; L.A. Stewart, Lancet, 2002, 359: 1011-1018). These doses also indicate that the SSKI was successful in largely blocking Iodine binding to the thyroid. Immnohistochemical studies in normal human tissues have failed to demonstrate TM-601 binding in normal thyroid gland (P. Kleihues and K. Webster, Eds. "Pathology and Genetics of Tumors of the Nervous System. Cavenee", International Agency for Research on Cancer, Lyon, 1997), which is consistent with our observation. Nonetheless, pre-blocking of the thyroid gland is likely important to minimize non-specific uptake of this agent.

### Patient follow-up, toxicity and response to therapy

Eleven of the 19 patients enrolled completed the 180 day observation period. There were no DLTs related to treatment during the initial 22-day observation period and no clinically significant acute adverse events during infusion of ¹³¹I-TM-601 at any dose level. The majority of events reported were mild to moderate in nature. ¹³¹I-TM-601 was well-tolerated by all patients. There were no grade III or IV toxicities related to the study drug or method of administration in the immediate and/or long-term follow up period. There were 88 grade I and 90 grade II toxicities. There were no patient complaints related to the study drug or method of administration.

Four patients had serious adverse events possibly or probably related to study medication reported within 22 days of administration (Table 5). These events included: one case of monoparesis and fatigue; one case of fever, chills, parasthesia in the upper extremities and brain edema; one case of infected intracavitary tumor site with osteomyelitis; and one case of headache related to pseudomeningocele. Additional serious adverse events reported beyond the initial 22-day observation period included one patient with generalized seizure and seizure with increased confusion; one patient with pneumonia; one patient with somnolence weakness, ventricular dilation and cerebral hematoma; one case of headache, face droopiness, dysathria and instability. The administration of a second dose of study medication was not associated with any serious adverse events although these events were not formally included in the toxicity evaluations due to the long time interval (12 and 19 weeks respectively) between drug administrations.

Over the course of the 180-day observation period, there were a total of seven deaths, all of which were reported in the 0.25 or 1.00 mg intracavitary ¹³¹I-TM-601 dosing groups. Two patients in Panel B with GBM have survived over 30 months. Median survival was 25.7 weeks for Panel 1 (0.25 mg dose); 77.6 weeks for Panel 2 (0.50 mg dose); 23.6 weeks for Panel 3 (1.00 mg dose) and 27.0 weeks for all three dosing groups.

**Table 5. Adverse Events**

| | SAE Grade | Event | No. Occurrences |
|---|---|---|---|
| All Events | 1 | Eye Pain | 1 |
| | | Nausea | 1 |
| | | Asthenia | 2 |
| | | Neck Pain | 1 |
| | | Aphasia | 1 |
| | | Hemiparesis | 3 |
| | | Parasthesia | 1 |
| | | Agitation | 1 |
| | | Anxiety | 1 |
| | | Depression | 1 |
| | | Meningocele repair | 1 |
| | 2 | Infection* | 1 |
| | | Altered liver function | 1 |
| | | Headache | 3 |
| | | Cerebral Edema | 1 |
| Events likely related to study drug | 1 | Infection | 1 |
| | | Paresis | 3 |
| | | Cerebral Edema | 1 |

| | | | |
|---|---|---|---|
| * one case each candidal infection and herpes zoster | | | |

### Radiographic changes

Tumor volume measurements were available for 16 patients at baseline. (within 48 hours of surgery), 16 patients at 22 days post-treatment, 16 patients at 90 days post-treatment, and 5 patients at 180 days post-treatment. All but one patient had some evidence of residual enhancing disease on initial post-operative scans. The mean post-operative residual T1 enhancing tumor volume was 28 ± 28cc (range 0-72.15cc). On day 22 post-treatment, this volume had increased to a mean of 31.8 ± 32.7cc (range 1.8-114.2cc). Mean changes (both increased and decreased sizes) in tumor (post contrast T1) volume from baseline to Day 22 were 0.8 cm³, 1.4 cm³, and 6.7 cm³ in the 0.25 mg, 0.5 mg and 1.0 mg intracavitary ¹³¹I-TM-601 dose groups, respectively (p values (for within treatment comparison) = 0.77, 0.31, and 0.74 respectively). The tumor volumes decreased by 10.8% in one patient and 76.7% in another were essentially unchanged in 9 patients, and increased significantly in 3 patients (4 patients were invaluable). This translated into a radiographic interpretation of stable disease in 12 patients and progressive disease in 4 patients (2 patients invaluable at this time point; Table 6).

For 16 patients with radiographic follow-up available at 90 days, a stable response was observed in 7 patients, and progressive disease in 9 patients (2 were not evaluable at this time point). Long-term follow-up was available for 6 patients, with I patient showing a partial response (PR; defined as at least 50% decrease under baseline with no new lesions), 4 with stable disease (SD), and 1 with progressive disease. Two patients (one SD, one PR) had stable radiographic disease for over 8 months and went on to a complete radiographic response (defined as complete absence of demonstrable contrast enhancement on T1 weighted MRI) without evidence of disease for 32 and 30 months. Both patients (#203 and #204) were females, ages 40-42. One patient had an initial diagnosis of anaplastic astrocytoma of the right parietal area which was grades a GBM on repeat resection, while the other patient had a recurrent GBM. Both patients had parietal lobe tumors (one left one right hemisphere), KPS of 90 after resection, and minimal residual enhancement on post-operative MRIs. Neither patient received a second dose of ¹³¹I-TM-601. An example of stable disease (patient #204) is demonstrated in FIG. 18D-E.

Changes in KPS from baseline varied slightly though Day 90 with little clinically significant data available at the end of the 180 day observation period.

In two GBM patients receiving 0.5 mg TM-601 + 10 (±10%) mCi ¹³¹I,a complete radiographic response-was observed. Those two patients are still alive 33 and 35 months after surgery (as of November 2005) even with this low dose of peptide and expected sub-thempeutic, level of radiation. Of note, these patients were slightly younger than the average patient in the study (ages 40 and 42 respectively), and had slightly higher KPS (90) than the average patient in the study, but were otherwise quite representative of the remainder of the study cohort.

**Table 6. Tumor response and survival**

| **Patient Number** | **Tumor response (MRI)** | | | **Survival (weeks)** |
|---|---|---|---|---|
| | **Day 22** | **Day 90** | **Day 180** | |
| 101 | PD* | PD | NA | 58.1 |
| 102. | SD | PD | NA | 25.0 |
| 103 | PD | NA | NA | 18.6 |
| 104 | SD | SD | SD | 26.4 |
| 105 | NA | PD | NA | 17.0 |
| 106 | SD | SD | NA | 27.2 |
| 201 | SD | SD | PD | 38.1 |
| 202 | NA | PD | NA | 44.9 |
| 203 | SD | SD | SD | 138.3 |
| 204 | SD | SD | PR | 133.7 |
| 205* | SD | SD | SD | 76.7 |
| 9206 | SD | PAD | NA | 78.6 |
| 301 | PD | PD | NA | 25.6 |
| 302 | SD | PD | NA | 21.9 |
| 303 | SD | SD | SD | 19.3 |
| 304 | SD | PD | NA | 25.4 |
| 305* | SD | PD | NA | 45.9 |
| 306 | PD | NA | NA | 20.1 |

| | | | | |
|---|---|---|---|---|
| *= Received second therapeutic dose of ¹³¹I-TM-601 on "compassionate use basins" | | | | |

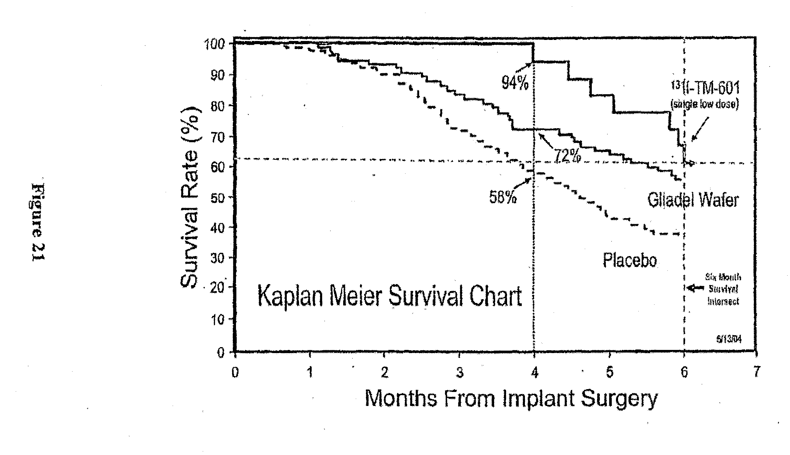

Although evaluation of efficacy was not the primary end-point of this study, tumor response and survival were monitored. Two patients in the 0.50 mg dose group experienced a minor response with a reduction greater than 50% in the product of axial diameters of the target tumor. Seven (39%) of the patients had stable disease over the' course of the 6 month follow-up and the nine (50%) patients had disease progression. There was no complete response observed. Overall, 8 patients were still alive upon the completion of the Day 180 follow-up and 5 patients survived longer than one year. As of February 2007, one patient remains alive, 4 years after recurrence. The 50% survival time was 5.7 months from the administration of treatment and 6.21 months from time of surgery and catheter placement in ¹³¹I-TM-601-treated patients. The survival of the patients enrolled in this study were pooled and compared to a historical group of patients with recurrent glioblastoma multiforme treated with. Gliadel^{®} wafers, as shown in FIG.21. Medium survival for this single low dose of 131I-TM-601 surpassed standard care and equaled that reported for Gliadel^{®} wafers in a historical group of patients.

In summary, treatment of patient with recurrent high-grade glioma with a single-dose of ¹³¹I-TM-601 administered in the surgical cavity was well tolerated up to the maximum dose tested of 1.0 mg and 10 mCi of ¹³¹I. The dose delivered to organs indicates that the dose of 131I could be increased substantially without harm to other organs and normal tissue, while still delivering a potentially therapeutic dose to the tumor cavity wall.

### EXAMPLE 18: Dose escalation and randomized multiple dose treatment of patients with high grade glioma with ¹³¹I-TM-601

The main objectives of this study was to determine the Maximum Tolerated Dose (MTD) of ¹³¹I-TM-601 administered intracavitary to patients with recurrent high-grade glioma; to determine the toxicity of a 3 and 6 dose cycle of ¹³¹I-TM-601 administrations into the tumor resection site of patients with recurrent high-grade glioma; to evaluate the 6- and 12-month rate of progression and survival of patients with recurrent high-grade glioma treated with a 3 or 6 dose cycle of ¹³¹I-TM-601; and to evaluate the overall time to progression and death of patients with recurrent high-grade gliomas treated with either a 3 or 6 dose cycle of ¹³¹I-TM-601. Other objectives include: to evaluate if either a 3 or 6 dose cycle of ¹³¹I-TM-601 affects Quality of Life; and to visualize the distribution of ¹³¹I within the tumor resection cavity and the surrounding brain after repeated intracavitary administration of ¹³¹I-TM-601, using brain SPECT images.

### Dose escalation Patients and Treatment Protocol

Fifteen (15) patients with recurrent glioblastoma multiforme (postoperatively), were enrolled in this sequential, multiple-dose escalation study with re-resection and implantation of a ventricular access device (VAD) into the resection cavity. Dose limiting toxicity was defined as any grade 3 or greater toxicity judged probably related to study drug and occurring within 7-11 days of the last dose. Dosing cohorts of 3-5 patients were entered at each of the dose levels listed in Table 7, designed to maintain a constant drug specific activity.

**Table 7: Sequential, multiple dose escalation study treatment groups**

| | A | B | C | D |
|---|---|---|---|---|
| Number of patients | 3 | 4 | 3 | 5 |
| Peptide(mg) | 0.4 | 0.6 | 0.8 | 0.8 |
| ¹³¹I (mCi) | 20 | 30 | 40 | 40 |
| Number of doses (weekly) | 3 | 3 | 3 | 6 |
| Total activity (mCi) | 60 | 90 | 120 | 240 |

Each patient may receive either 3 or 6 injections (to the surgical cavity through a VAD) of ¹³¹I-1M-601 given at 7 days intervals (unless dose is delayed for up to 28 days due to toxicity, safety issue or major scheduling problem). A dose regiment utilizing administration every 7 days is advantageous because by this time point normal organs have substantially cleared the ¹³¹-TM-602, while the tumor still has an appreciable amount of radioactivity present, as shown in Table 3.

### Patient follow-up, toxicity and response to therapy

One subject did not complete treatment due to thrombocytopenia possibly related to a VAD infection necessitating removal of the VAD following the initial dose of study drug. Thirteen serious adverse events (SAEs) were observed in 15 patients after study drug administration. These included seizure, cerebral edema, and gait disturbances. None were judged to be both unexpected and related to study drug. Five SAEs were judged to be possibly related to ¹³¹I-chlorotoxin, including 3 seizures, 1 cerebral edema, and 1 thrombocytopenia (CTC grade 3). Two subjects experienced Grade 1/2 CTC 3.0 neutropenia.

No dose limiting toxicity was observed. The safety profile of weekly intracavitary injections of ¹³¹I-chlorotoxin was acceptable. Dose limiting toxicity was not reached at the final planned dose level D (6 x 40 mCi/0.8 mg peptide, total 240 mCi)(MPD), which was determined the maximum dose.

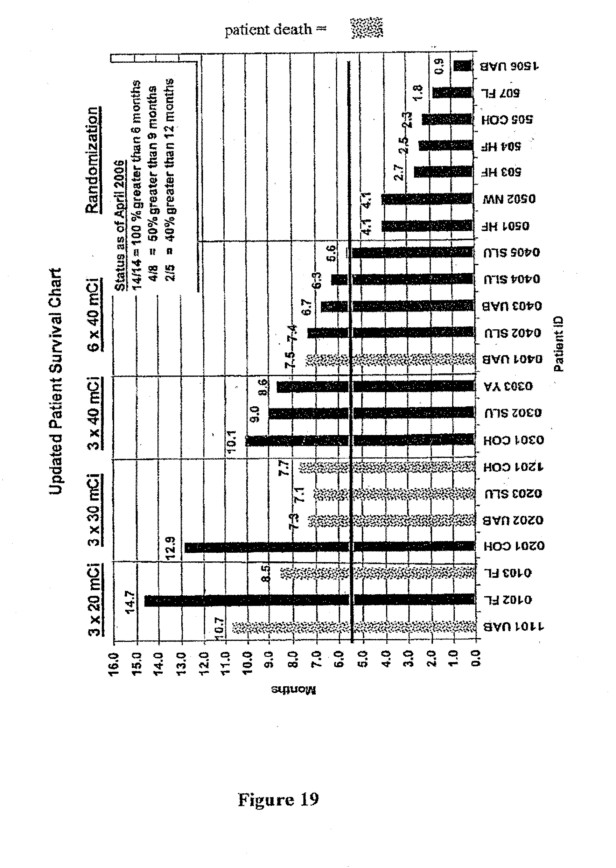

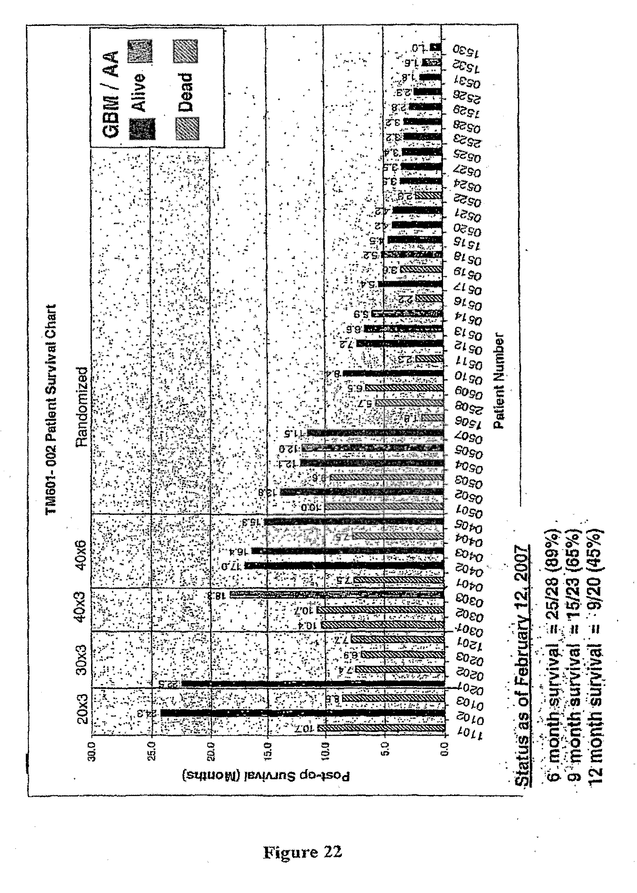

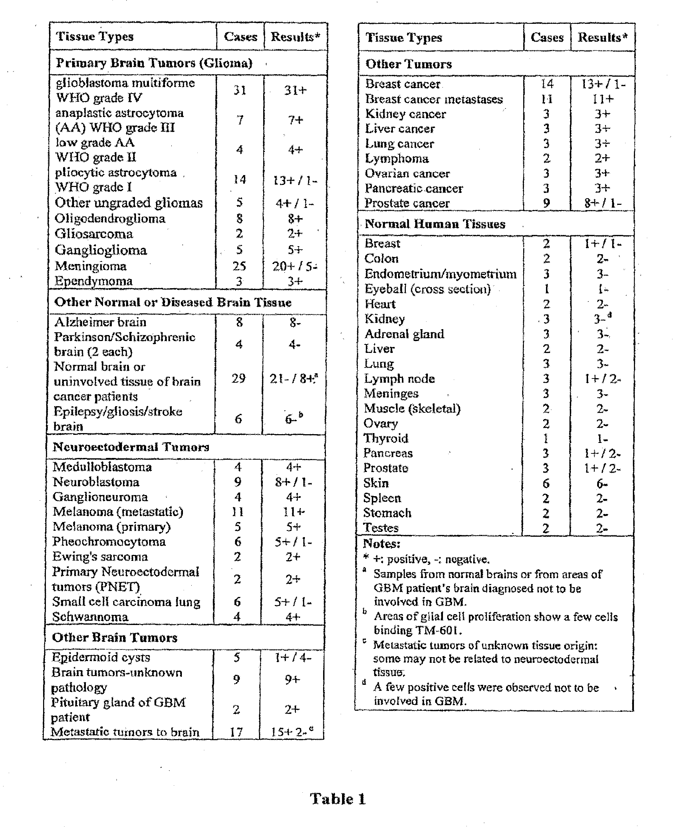

FIG. 19 and FIG. 22 depict survival data results for individual patients as of April 2006, and as of February 2007, respectively.

### Randomized Study

Following studies to determine dose limiting toxicity (DLT), maximum tolerated dose (MTD), and maximum practical dose (MPD), malignant glioma patients were enrolled in a randomized study to compare safety, time to disease progression and survival rates following treatment. Malignant glioma patients enrolled are randomly assigned to Treatment Groups receiving three or six injections of ¹³¹I-TM-601 at the MPD. Since DLT was not reached at the final planned dose, patients enrolled will undergo re-resection and implantation of a ventricular access device (VAD) into the resection cavity, and receive either (a) ¹³¹I-TM-601 at 0.8mg/40mCi, repeated 3 times at 7 day intervals (Group A) or (b) ¹³¹I-TM-601 at 0.8mg/40mCi, repeated 6 times at 7 day intervals (Group B).

27 patients are planned to be enrolled in each of Group A and Group B. As of February 2007, 32 patients have been enrolled that are evaluable (*i.e*., that have received 1 or more injections).

Patients enrolled in the randomized arm of the study are continuously being monitored and are being assessed by physical and neurological examinations, blood chemistry and platelet analyses, thyroid function tests, ECG, monitoring of adverse events (and treatment where necessary), treatment with concomitant medications, KPS, MRI evaluation, evaluation of disease progression, and patient survival.

FIG 19 and FIG. 22 depict survival data results for individual patients as of April 2006, and as of February 2007, respectively. As of February 2007, in the dose escalation and randomized studies, the 6-month survival is 89% (25 out of 28 patients), the 9 month survival is 65% (15 out of 23 patients) and the 12-month survival is 4.5% (9 out of 20 patients).

### Imaging and Dosimetry Study

Patients randomly assigned to Treatment Group receiving six injections of 0.8 mg of ¹³¹I-TM-601 with 40mCi ¹³¹I (± 10%) are eligible for participation in a sub-study to evaluate and compare the biodistribution (% injection dose) and clearance of ¹³¹I in the resected tumor cavity and normal organs with uptake of ¹³¹I-TM-601 following the first, third, and sixth doses. Patients participating in the study will undergo monitoring imaging procedures described in Table 8.

Biodistribution, % injection dose (% ID) and clearance of ¹³¹I. after single and multiple doses of ¹³¹I-TM-601, in normal organs with significant uptake of ¹³¹I will be determined using serial, quantitative planar images. Anterior and posterior whole-body images of ¹³¹I, after single and multiple doses of ¹³¹I-TM-601 will be acquired on a dual-detector camera equipped with high-energy collimator. Images will be acquired immediately (Day 1) and at Days 2, 3, 4 and at one time between Day 6-8. The energy window will be centered at 364 keV and the image matrix will be 1024 x 256 with a scan speed of 10 cm/min. A 20 mL calibrated ¹³¹I source of 100 µCi will be placed on the imaging table 10 cm away from the patient's feet during the whole-body scan. Any residue amount of ¹³¹I in the syringe should be recorded to determine the net injection dose of ¹³¹I-TM-601 to the patient.

**Table 8: Procedures for Dosimetry Study**

| **Procedure** | **To be done for first, third and sixth ¹³¹I-TM-601 injections** | | | | | |
|---|---|---|---|---|---|---|
| | **Days** (Day 1 is dosing day) | | | | | |
| | 1 | 2 | | 3 | 4 | 6-8 (i) |
| ⁵⁰Co Body Nuclear Scan | X (a) | | | | | |
| Brain MRI Scan | X (b) | | | | | |
| ¹³¹I-TM-601 Body Nuclear Scan | X(c) | X(g) | | X(g) | X (g) | X(g,j) |
| Brain SPECT (d) | X(c) | X(g) | | X(g) | X(g) | X(g,j) |
| Blood Radioactivity Evaluation | X(e) | X(h) | | X(h) | X(h) | X(h) |
| Urine Radioactivity Evaluation | X(f) | X(i) | | X(i) | X (i) | X(i) |
| a) Prior to VAD potency verification (first ¹³¹I-TM-601 dose only) | | | f) 24 hour urine collection- (initiate immediately post infusion). | | | |
| b) Performed day following surgery/ VAD placement, or as soon as judged clinically feasible by the P.I. Place 3 fiducial markers for image fusion. | | | g) Done same time as Day 1. | | | |
| c) Performed immediately after ¹³¹I-TM-601 infusion is complete. | | | h) Collect at time of imaging. | | | |
| d) Place 3 fiducial markers for image dosing. | | | i) Urine collection since last visit. | | | |
| e) Collect prior to ¹³¹I-TM-601 injection and at 1,2 and 4 hours post infusion. | | | j) If done on day dosing,must be done prior to next dose. | | | |

Whole blood and urine samples will be collected as described in Table 8. Briefly, whole blood samples will be collected prior to ¹³¹I-TM-601 injection and at 1,2 and 4 hours post injections (Day 1) and at the time of imaging on Days 2, 3, 4 and at the time of imaging between Days 6-8 prior to each subsequent ¹³¹I-TM-601 injection. Whole blood and urine samples will be collected only for the first, third and sixth ¹³¹I-TM-601 injections. One mL of whole blood will be placed in a test tube and counted using a gamma well counter. The whole blood sample counts will be converted to µCi of ¹³¹I using a calibrated ¹³¹I standard source.

### EXAMPLE 19: In vitro Binding of Chlorotoxin Labeled with ^{99m}Tc

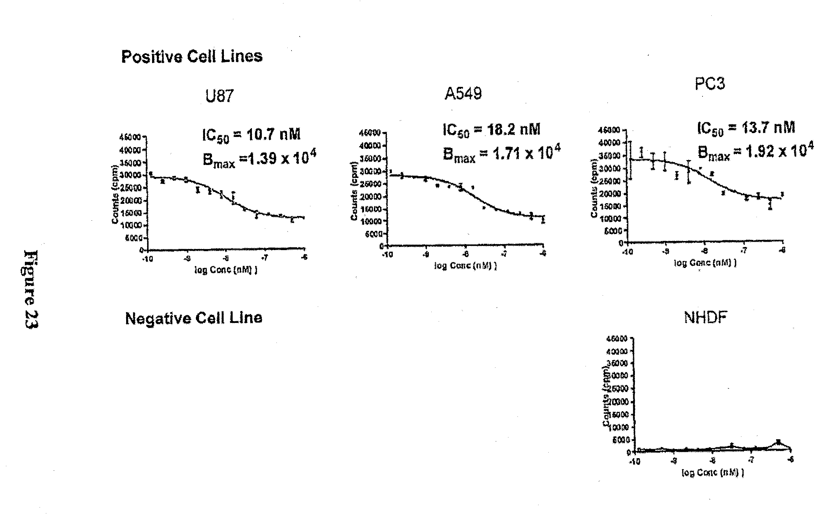

FIG. 23 presents binding curves that were obtained for ^{99m}Tc-labeled chlorotoxin incubated in normal human cells (NHDF: normal human dermal fibroblasts) and in human tumor cells: U87 cells (a malignant glioma cell line), A549 cells (lung epithelial carcinoma cell line) and PC3 cells (prostate cancer cell line). The results clearly show that ^{99m}Tc-labeled chlorotoxin binds to human tumor cells but not to human normal cells.

### Other Embodiments

Other embodiments of the invention will be apparent to those skilled in the art from a consideration of the specification or practice of the invention disclosed herein. It is intended that the specification and examples be considered as exemplary only, with the true scope of the invention being indicated by the following claims.

## Claims

1. A cytotoxic chlorotoxin conjugate for use in treating an individual having a neuroectodermally-derived tumour, wherein the cytotoxic chlorotoxin conjugate comprises at least one chlorotoxin moiety associated with at least one cytotoxic moiety and wherein the chlorotoxin conjugate is provided in a dose containing between approximately 0.1 mg and approximately 5.0 mg of chlorotoxin moiety.

2. The chlorotoxin conjugate of claim 1, wherein the neuroectodermally-derived tumour is a member of the group consisting of glioma, meningioma, ependymoma, medulloblastoma, neuroblastoma, ganglioma, pheochromocytoma, melanoma, peripheral primitive neuroectodermal tumour, small cell carcinoma of the lung, Ewing's sarcoma, and metastatic tumour of neuroectodermal origin in the brain or wherein the neuroectodermally-derived tumour is a refractory tumour.

3. The chlorotoxin conjugate of claim 1, wherein the neuroectodermally-derived tumour is located in the brain, preferably wherein the neuroectodermally-derived tumour is a glioma, such as a high-grade glioma, preferably wherein the high-grade glioma is a recurrent high-grade glioma.

4. The chlorotoxin conjugate of claim 1, wherein the cytotoxic moiety comprises a radioisotope, such as iodine-131(¹³¹I).

5. The chlorotoxin conjugate of claim 3, wherein at least one dose of cytotoxic chlorotoxin conjugate is administered by intracavitary administration.

6. The chlorotoxin conjugate of claim 5, wherein the dose of cytotoxic chlorotoxin conjugate contains approximately 0.25 mg of chlorotoxin moiety, approximately 0.4 mg of chlorotoxin moiety, approximately 0.5 mg of chlorotoxin moiety, approximately 0.6 mg of chlorotoxin moiety, approximately 0.8 mg of chlorotoxin moiety, approximately 1.0 mg of chlorotoxin moiety, approximately 2.0 mg of chlorotoxin moiety, approximately 3.0 mg of chlorotoxin moiety, approximately 4.0 mg of chlorotoxin moiety, or approximately 5.0 mg of chlorotoxin moiety.

7. The chlorotoxin conjugate of claim 5, wherein the cytotoxic chlorotoxin conjugate comprises ¹³¹I and the dose of cytotoxic chlorotoxin conjugate contains between approximately 10 mCuries and approximately 50 mCuries of ¹³¹I.

8. The chlorotoxin conjugate of claim 5, wherein the at least two doses of cytotoxic chlorotoxin conjugate is administered by intracavitary administration.

9. The chlorotoxin conjugate of claim 8, wherein two consecutive does of cytotoxic chlorotoxin conjugate are administered 1 week apart.

10. The chlorotoxin conjugate of claim 1 further comprising a chemotherapeutic agent.

11. A method of differentiating neuroectodermally-derived neoplastic tumour tissue from non-neoplastic tissue, the method comprising the steps of:
contacting a tissue of interest with a diagnostic chlorotoxin agent, wherein the diagnostic chlorotoxin agent comprises at least one chlorotoxin moiety associated with at least one labelling moiety; and
measuring binding of the diagnostic chlorotoxin agent to the tissue of interest, wherein an elevated level of binding, relative to normal tissue, indicates that the tissue of interest is a neuroectodermally-derived tumour tissue.

12. The method of claim 11, wherein the step of measuring binding of the diagnostic chlorotoxin agent to the tissue of interest is performed using a technique selected from the group consisting of fluorescence microscopy, bioluminescence, fluorescent activated cell sorting (FACS), histochemical staining, ELISA, Magnetic Resonance Imaging (MRI), Gamma camera, Single Photon Emission Computed Tomography (SPECT) and Positron Emission Tomography (PET).

13. The method of claim 11, wherein the tissue of interest originates from an individual suspected of having a neuroectodermally-derived tumour, preferably wherein the neuroectodermally-derived tumour is a member of the group consisting of glioma, meningioma, ependymoma, medulloblastoma, neuroblastoma, ganglioma, pheochromocytoma, melanoma, peripheral primitive neuroectodermal tumour, small cell carcinoma of the lung, Ewing's sarcoma, and metastatic tumour of neuroectodermal origin in the brain.

14. The chlorotoxin conjugate of claim 6, wherein at least two additional doses, at least five additional doses or more than five additional doses of cytotoxic chlorotoxin conjugate is administered by intracavitary administration, which additional doses each contains approximately 0.25 mg of chlorotoxin moiety, approximately 0.4 mg of chlorotoxin moiety, approximately 0.5 mg of chlorotoxin moiety, approximately 0.6 mg of chlorotoxin moiety, approximately 0.8 mg of chlorotoxin moiety, approximately 1.0 mg of chlorotoxin moiety, approximately 2.0 mg of chlorotoxin moiety, approximately 3.0 mg of chlorotoxin moiety, approximately 4.0 mg of chlorotoxin moiety, or approximately 5.0 mg of chlorotoxin moiety.
